# EUROPEAN PATENT SPECIFICATION

(11) **EP 1 240 166 B1**
(45) Date of publication and mention of the grant of the patent: **16.02.2005**
(21) Application number: 00990204.0
(22) Date of filing: 07.12.2000
(51) Int. Cl.: C07D 417/12, C07D 417/14, A61K 31/427, A61K 31/454, A61P 35/00

(54) **N-[5-[[[5-ALKYL-2-OXAZOLYL]METHYL]THIO]-2-THIAZOLYL CARBOXAMIDE INHIBITORS OF CYCLIN DEPENDENT KINASES**
N-[5-[[[5-ALKYL-2-OXAZOLYL]METHYL]THIO]-2-THIAZOLYL CARBOXAMIDE ALS INHIBITOREN CYCLINABHÄNGIGER KINASEN
INHIBITEURS CONTENANT N-[5-[[[5-ALKYL-2-OXAZOLYL]METHYL]THIO]-2-THIAZOLYL]CARBOXAMIDE INHIBANT DES KINASES DEPENDANTES DES CYCLINES

(30) Priority: 15.12.1999 US 464511; 26.07.2000 US 616627
(43) Date of publication of application: 18.09.2002
(73) Proprietor: BRISTOL-MYERS SQUIBB COMPANY, Princeton, NJ 08543-4000 (US)
(72) Inventor: MISRA, Raj, N., Hopewell, NJ 08525 (US); XIAO, Hai-Yun, Princeton, NJ 08540 (US)
(74) Representative: Hubert, Philippe
(86) International application number: PCT/US2000/033501
(87) International publication number: WO 2001/044242

(56) References cited:
- WO-A-99/24416

## Description

### Brief Description of the Invention

The present invention is directed to compounds of formula I 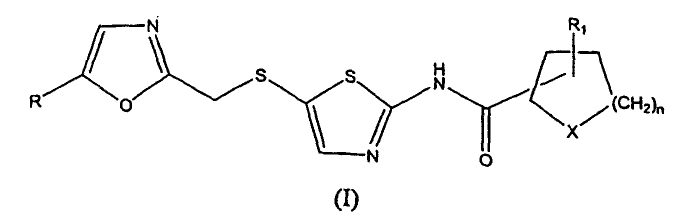 and enantiomers, diastereomers and pharmaceutically acceptable salts thereof wherein R is t-butyl;
R₁ is hydrogen or alkyl;
X is NR₂ or CHNR₂R₃;
R₂ and R₃ are each independently hydrogen, alkyl, substituted alkyl, cycloalkyl or substituted cycloalkyl; and
n is 0, 1, 2 or 3.

The compounds of formula I are particularly useful as potent, protein kinase inhibitors and are useful in the treatment of proliferative diseases, for example, cancer, inflammation and arthritis. They may also be useful in the treatment of Alzheimer's disease, and cardiovascular disease.

WO-99/24416 discloses aminothiazole compounds, for which cyclin-dependent kinase-inhibition properties are claimed. The compounds according to WO-99/24416 vary in structure, but all contain a central 2-amino, 5-thio-substituted aminothiazole ring (where the 5-thio substituent may optionally be oxidized to the sulfoxide or sulfone level). As represented by general formula (I) of WO-99/24416, the chain connected to the aminothiazole ring via its 5-thio substituent terminates in an aryl or heteroaryl group.

### Description of the Invention

The present invention provides for compounds of formula I, pharmaceutical compositions employing such compounds, and for methods of using such compounds.

Listed below are definitions of various terms used to describe the compounds of the instant invention. These definitions apply to the terms as they are used throughout the specification (unless they are otherwise limited in specific instances) either individually or as part of a larger group.

The term "alkyl" or "alk" refers to a monovalent alkane (hydrocarbon) derived radical containing from 1 to 12, preferably 1 to 6, and more preferably 1 to 4, carbon atoms unless otherwise defined. An alkyl group is an optionally substituted straight, branched or cyclic saturated hydrocarbon group. When substituted, alkyl groups may be substituted with up to four substituent groups, R₄ as defined, at any available point of attachment. When the alkyl group is said to be substituted with an alkyl group, this is used interchangeably with "branched alkyl group". Exemplary unsubstituted such groups include methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, and the like. Exemplary substituents may include, but are not limited to, one or more of the following groups: halo (such as F, Cl, Br or I), haloalkyl (such as CCl₃ or CF₃), alkoxy, alkylthio, hydroxy, carboxy, alkylcarbonyl, alkyloxycarbonyl, alkylcarbonyloxy, amino, carbamoyl, urea, amidinyl, or thiol.

Cycloalkyl is a specie of alkyl containing from 3 to 15 carbon atoms, without alternating or resonating double bonds between carbon atoms. It may contain from 1 to 4 rings. Exemplary unsubstituted such groups include cyclopropyl, cyclobutyl, cyclopentyl, cyclohexyl, etc. Exemplary substituents include one or more of the following groups: halogen, alkyl, alkoxy, alkyl hydroxy, amino, nitro, cyano, thiol and/or alkylthio.

The terms "alkoxy" or "alkylthio", as used herein, denote an alkyl group as described above bonded through an oxygen linkage (-O-) or a sulfur linkage (-S-), respectively.

The term "alkyloxycarbonyl", as used herein, denotes an alkoxy group bonded through a carbonyl group. An alkoxycarbonyl radical is represented by the formula: -C(O)OR₅, where the R₅ group is a straight or branched C₁₋₆ alkyl group.

The term "alkylcarbonyl" refers to an alkyl group bonded through a carbonyl group.

The term "alkylcarbonyloxy", as used herein, denotes an alkylcarbonyl group which is bonded through an oxygen linkage.

Pharmaceutically acceptable salts of compounds of formula I which are suitable for use in the methods and compositions of the present invention include, but are not limited to, salts formed with a variety of organic and inorganic acids such as hydrogen chloride, hydroxymethane sulfonic acid, hydrogen bromide, hydrogen iodide, methanesulfonic acid, sulfuric acid, acetic acid, trifluoroacetic acid, maleic acid, fumaric acid, benzenesulfonic acid, toluenesulfonic acid and various others, e.g., nitrates, phosphates, borates, tartarates, citrates, succinates, benzoates, ascorbates, salicylates, and the like. These salts include racemic forms as well as enantiomers, and diastereomers (such as, for example, D-tartarate and L-tartarate salts). In addition, pharmaceutically acceptable salts of compounds of formula I may be formed with alkali metals such as sodium, potassium and lithium; alkaline earth metals such as calcium and magnesium; organic bases such as dicyclohexylamine, tributylamine, and pyridines, and the like; and amino acids such as arginine, lysine and the like.

All stereoisomers of the compounds of the instant invention are contemplated, either in admixture or in pure or substantially pure form. The definition of the compounds according to the invention embraces all possible stereoisomers and their mixtures. It very particularly embraces the racemic forms and the isolated optical isomers having the specified activity. The racemic forms can be resolved by physical methods, such as, for example, fractional crystallization, separation or crystallization of diastereomeric derivatives or separation by chiral column chromatography. The individual optical isomers can be obtained from the racemates by conventional methods, such as, for example, salt formation with an optically active acid followed by crystallization.

All configurational isomers of compounds of the present invention are contemplated, either in admixture or in pure or substantially pure form. The definition of compounds of the present invention very particularly embraces both cis and trans isomers of cycloalkyl rings.

In the context of the present invention, the definition of compounds of the present invention includes the free base, enantiomers, diastereomers as well as pharmaceutically acceptable salts. Examples of such pharmaceutically acceptable salts include, but are not limited to, hydrochloride, dihydrochloride, sulfate, trifluoroacetate, mixture of trifluoroacetate and hydrochloride, tartarate, fumarate, succinate, maleate, citrate, methanesulfonate, bromate and iodate salts. Also included are salts formed with other organic and inorganic acids such as hydroxymethane sulfonic acid, acetic acid, benzenesulfonic acid, toluenesulfonic acid and various others, e.g., nitrates, phosphates, borates, benzoates, ascorbates, salicylates, and the like. These salts include racemic forms as well as enantiomers and diastereomers (such as, for example, D-tartarate and L-tartarate salts). In addition, pharmaceutically acceptable salts of the formula I compounds may be formed with alkali metals such as sodium, potassium and lithium; alkaline earth metals such as calcium and magnesium; organic bases such as dicyclohexylamine, tributylamine, and pyridines, and the like; and amino acids such as arginine, lysine and the like.

It should be understood that solvates (e.g. hydrates) of the compounds of formula I are also within the scope of the present invention. Methods of solvation are generally known in the art. Accordingly, the compounds of the instant invention may be in the free or hydrate form, and may be obtained by methods exemplified by the following schemes.

Compounds of formula I may generally be prepared, as shown in Scheme 1, by reacting an amine of formula II with a carboxylic acid of formula III in the presence of 1-(3-dimethylaminopropyl)-3-ethylcarbodiimide hydrochloride and a base.

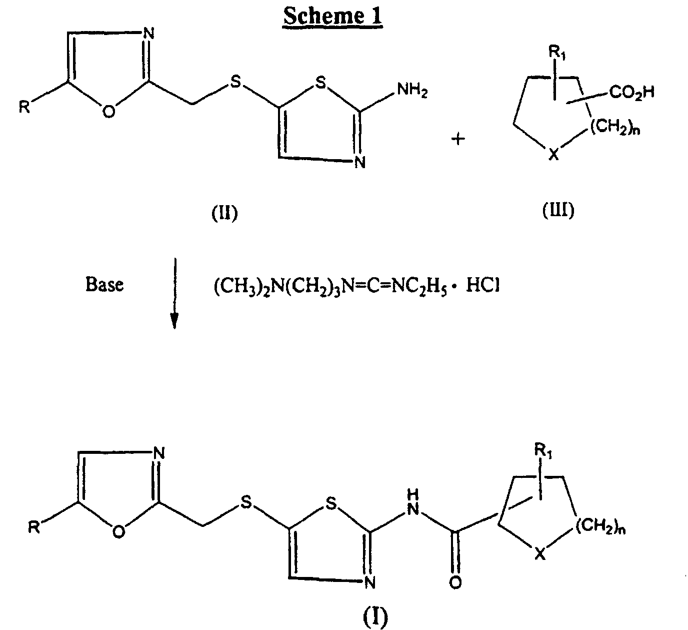

Formula I compounds wherein X is NR₂ and R₂ is hydrogen may be prepared, as shown in Scheme 2, by reacting an amine of formula II with a carboxylic acid of formula IV in the presence of 1-(3-dimethylaminopropyl)-3-ethylcarbodiimide hydrochloride and a base to form an N-protected compound of formula V, and deprotecting the formula V compound with acid.

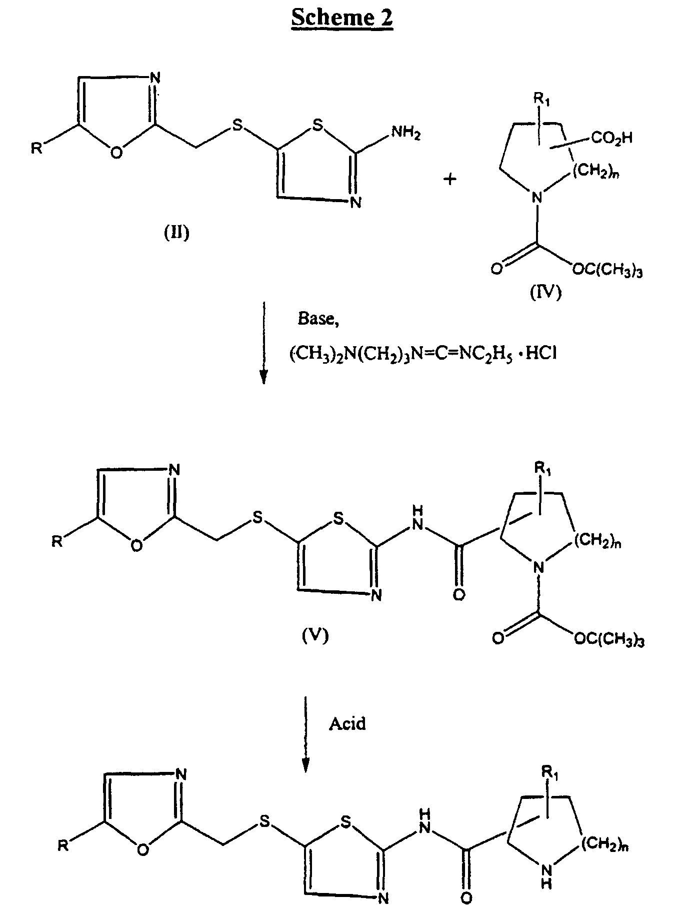

Compounds of formula I wherein X is NR₂ and R₂ is 2,3-dihydroxypropyl may be prepared, as shown in Scheme 3, by reacting a compound of formula I wherein X is NR2 and R₂ is hydrogen with glyceraldehyde in the presence of a reducing agent such as sodium triacetoxyborohydride and an alcohol such as methanol.

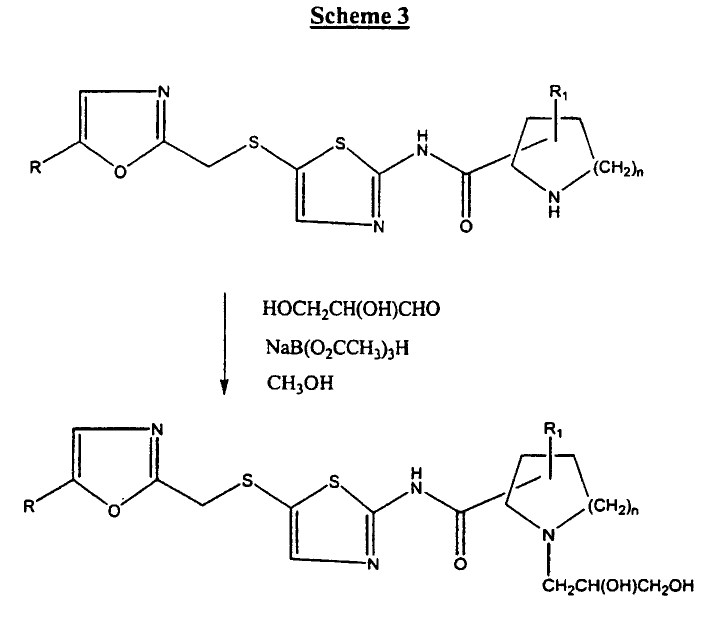

Formula I compounds wherein X is NR₂ and R₂ is 2-hydroxyethyl may be prepared, as shown in Scheme 4, by reacting a compound of formula I wherein X is NR₂ and R₂ is hydrogen with a 2-(bromoethoxy)trialkylsilane of formula VI to form an intermediate compound of formula VII, and deprotecting the formula VI compound with an acid such as hydrogen fluoride.

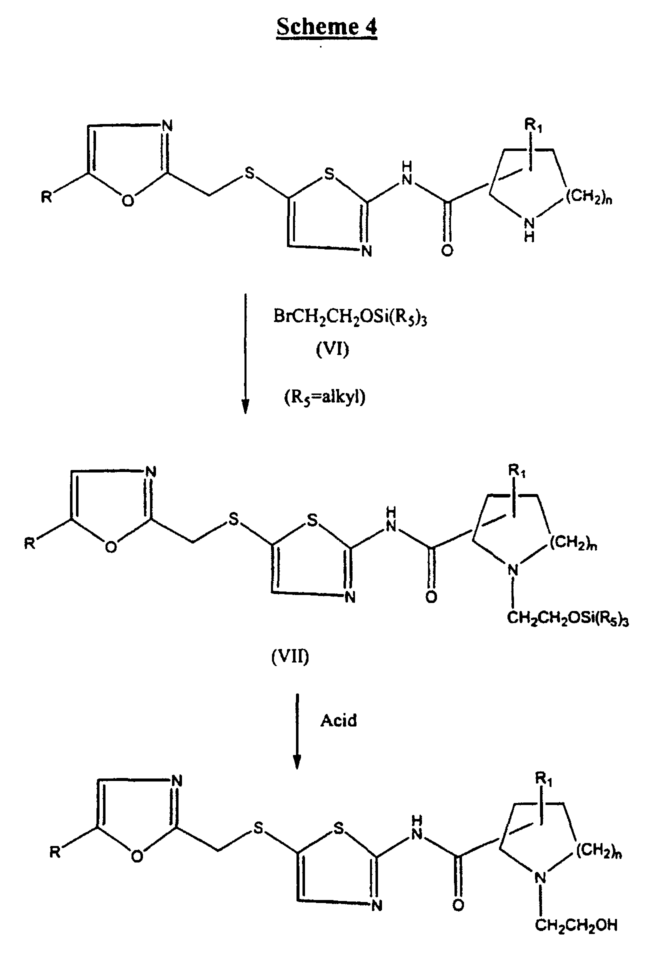

Starting amines of formula II may be prepared as shown in Scheme 5. An alpha-bromoketone of formula VIII may be reacted with sodium azide in a solvent such as dimethylformamide to provide the azido ketone derivative IX, which is reduced by a reducing agent such as hydrogen in the presence of palladium on carbon catalyst, or triphenylphosphine to provide the amino ketone X. Compound X may alternatively be prepared by reaction of the alpha-bromoketone of formula VIII with hexamethylenetetramine in a solvent such as acetone to give the compound of formula XI, which is hydrolyzed by an acidic medium such as hydrochloric acid in ethanol. Compounds of formula X may be acylated by an agent such as 2-chloroacetyl chloride to provide amides of formula XII. The formula XII amides are cyclized to 2-chloromethyl oxazoles of formula XIII using a dehydrating agent such as phosphorous oxychloride in toluene. Reaction of the chloromethyl oxazoles of formula XIII with thiourea in a solvent such as ethanol provides the thiourea derivatives XIV, which may be reacted with 5-bromo-2-aminothiazole in the presence of a base such as potassium hydroxide in alcohol to give formula II amines. Alternatively, reaction of the chloromethyl oxazole derivatives of formula XIII with 5-thiocyano-2-aminothiazole, in the presence of a reducing agent such as sodium borohydride, provides compounds of formula II.

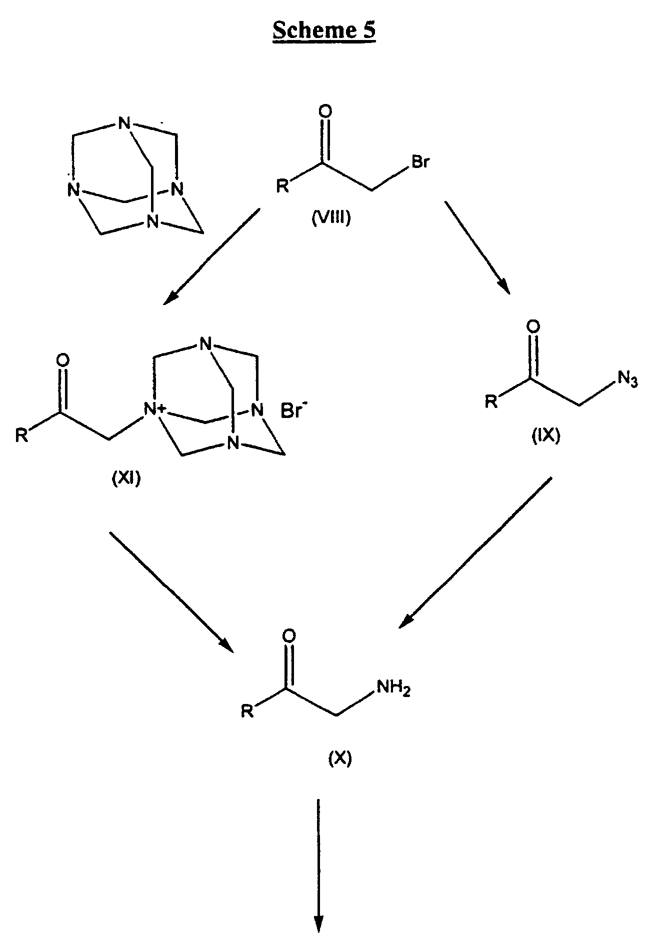

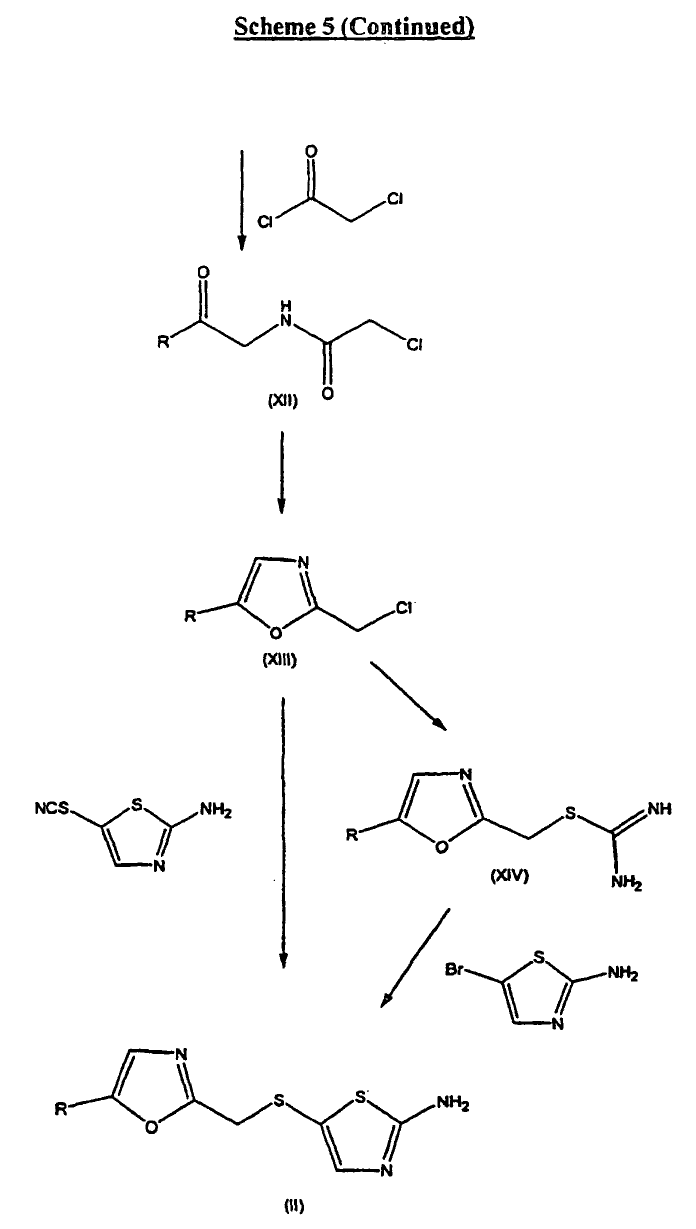

Preferred compounds of formula I are those wherein:
R is t-butyl;
R₁ is hydrogen;
X is NR₂ or CHNR₂R₃;
R₂ and R₃ are each independently hydrogen, alkyl, substituted alkyl or cycloalkyl; and
n is 2.

A first group of more preferred compounds of the present invention are those of formula Ia 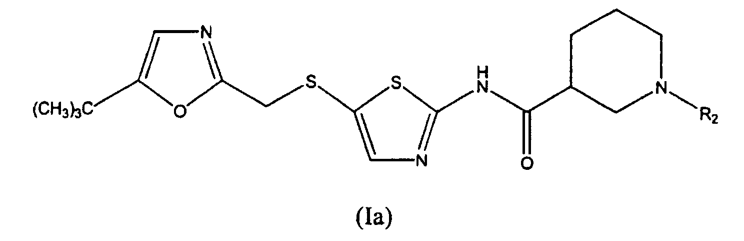 and enantiomers, diastereomers and pharmaceutically acceptable salts thereof wherein R₂ is hydrogen, alkyl, substituted alkyl or cycloalkyl.

A second group of more preferred compounds of this invention are those of formula Ib 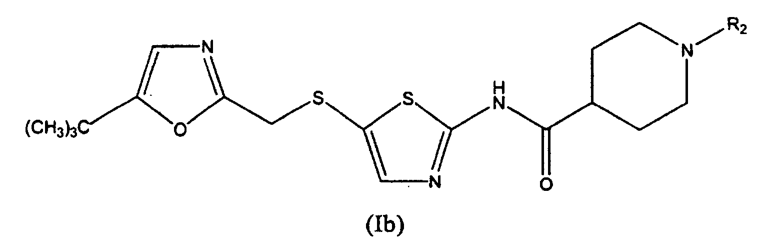 and enantiomers, diastereomers and pharmaceutically acceptable salts thereof wherein R₂ is hydrogen, alkyl, substituted alkyl or cycloalkyl.

A third group of more preferred compounds of the present invention are those of formula Ic 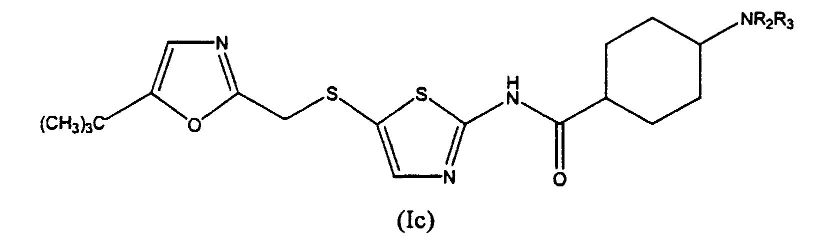 and enantiomers, diastereomers and pharmaceutically acceptable salts thereof wherein R₂ and R₃ are each independently hydrogen, alkyl, substituted alkyl or cycloalkyl.

Formula I compounds particularly useful in the methods of this invention include:
N-[5-[[[5-(1,1-dimethylethyl)-2-oxazolyl]methyl]thio]-2-thiazolyl]-4-piperidinecarboxamide;
(±)-N-[5-[[[5-(1,1-dimethylethyl)-2-oxazolyl]methyl]thio]-2-thiazolyl]-3-piperidinecarboxamide;
(±)-1-(2,3-dihydroxypropyl)-N-[5-[[[5-(1,1-dimethylethyl)-2-oxazolyl]-methyl]thio]-2-thiazolyl]-4-piperidinecarboxamide;
N-[5-[[[5-(1,1-dimethylethyl)-2-oxazolyl]methyl]thio]-2-thiazolyl]-1-(1-methylethyl)-4-piperidinecarboxamide;
1-cyclopropyl-N-[5-[[[5-(1,1-dimethylethyl)-2-oxazolyl]methyl]thio]-2-thiazolyl]-4-piperidinecarboxamide;
N-[5-[[[5-(1,1-dimethylethyl)-2-oxazolyl]methyl]thio]-2-thiazolyl]-1-(2-hydroxyethyl)-4-piperidinecarboxamide;
(R)-N-[5-[[[5-(1,1-dimethylethyl)-2-oxazolyl]methyl]thio]-2-thiazolyl]-3-piperidinecarboxamide;
(S)-N-[5-[[[5-(1,1-dimethylethyl)-2-oxazolyl]methyl]thio]-2-thiazolyl]-3-piperidinecarboxamide;
cis-4-amino-N-[5-[[[5-(1,1-dimethylethyl)-2-oxazolyl]methyl]thio]-2-thiazolyl]cyclohexylcarboxamide; and
trans-4-amino-N-[5-[[[5-(1,1-dimethylethyl)-2-oxazolyl]methyl]thio]-2-thiazolyl]cyclohexylcarboxamide; and
pharmaceutically acceptable salts thereof.

The present invention also includes methods based upon the pharmacological properties of the compounds of the invention. It should be noted that, in the context of the methods of the present invention, the compounds of the invention, or compounds of formula I, refer to the free base, enantiomers, diastereomers as well as pharmaceutically acceptable salts. Examples of such pharmaceutically acceptable salts include, but are not limited to, hydrochloride, dihydrochloride, sulfate, trifluoroacetate, mixture of trifluoroacetate and hydrochloride, tartarate, fumarate, succinate, maleate, citrate, methanesulfonate, bromate and iodate salts. Also included are salts formed with other organic and inorganic acids such as hydroxymethane sulfonic acid, acetic acid, benzenesulfonic acid, toluenesulfonic acid and various others, e.g., nitrates, phosphates, borates, benzoates, ascorbates, salicylates, and the like. These salts include racemic forms as well as enantiomers and diastereomers (such as, for example, D-tartarate and L-tartarate salts). In addition, pharmaceutically acceptable salts of compounds of formula I maybe formed with alkali metals such as sodium, potassium and lithium; alkaline earth metals such as calcium and magnesium; organic bases such as dicyclohexylamine, tributylamine, and pyridines, and the like; and amino acids such as arginine, lysine and the like.

The compounds according to the invention have pharmacological properties; in particular, the compounds of formula I are inhibitors of protein kinases such as the cyclin dependent kinases (cdks), for example, cdc2 (cdk1), cdk2, cdk3, cdk4, cdk5, cdk6, cdk7 and cdk8. The novel compounds of formula I are expected to be useful in the therapy of proliferative diseases such as cancer, inflammation, arthritis, Alzheimer's disease and cardiovascular disease. These compounds may also be useful in the treatment of topical and systemic fungal infections.

More specifically, the compounds of formula I are useful in the treatment of a variety of cancers, including (but not limited to) the following:
- carcinoma, including that of the bladder, breast, colon, kidney, liver, lung, ovary, pancreas, stomach, cervix, thyroid, prostate, and skin;
- hematopoietic tumors of lymphoid lineage, including acute lymphocytic leukemia, B-cell lymphoma, and Burkett's lymphoma;
- hematopoietic tumors of myeloid lineage, including acute and chronic myelogenous leukemias and promyelocytic leukemia;
- tumors of mesenchymal origin, including fibrosarcoma and rhabdomyosarcoma; and
- other tumors, including melanoma, seminoma, teratocarcinoma, osteosarcoma, neuroblastoma and glioma.

Due to the key role of cdks in the regulation of cellular proliferation in general, inhibitors could act as reversible cytostatic agents which may be useful in the treatment of any disease process which features abnormal cellular proliferation, e.g., neuro-fibromatosis, atherosclerosis, pulmonary fibrosis, arthritis, psoriasis, glomerulonephritis, restenosis following angioplasty or vascular surgery, hypertrophic scar formation, inflammatory bowel disease, transplantation rejection, angiogenesis, and endotoxic shock.

Compounds of formula I may also be useful in the treatment of Alzheimer's disease, as suggested by the recent finding that cdk5 is involved in the phosphorylation of tau protein (*J*. *Biochem,* 117, 741-749 (1995)).

Compounds of formula I may also act as inhibitors of other protein kinases, e.g., protein kinase C, her2, rafl, MEK1, MAP kinase, EGF receptor, PDGF receptor, IGF receptor, PI3 kinase, weel kinase, Src, Abl, VEGF, and lck, and thus be effective in the treatment of diseases associated with other protein kinases.

Compounds of formula I also induce or inhibit apoptosis, a physiological cell death process critical for normal development and homeostasis. Alterations of apoptotic pathways contribute to the pathogenesis of a variety of human diseases. Compounds of formula I, as modulators of apoptosis, will be useful in the treatment of a variety of human diseases with abberations in apoptosis including cancer (particularly, but not limited to, follicular lymphomas, carcinomas with p53 mutations, hormone dependent tumors of the breast, prostate and ovary, and precancerous lesions such as familial adenomatous polyposis), viral infections (including, but not limited to, herpesvirus, poxvirus, Epstein-Barr virus, Sindbis virus and adenovirus), autoimmune diseases (including, but not limited to, systemic lupus, erythematosus, immune mediated glomerulonephritis, rheumatoid arthritis, psoriasis, inflammatory bowel diseases, and autoimmune diabetes mellitus), neurodegenerative disorders (including, but not limited to, Alzheimer's disease, AIDS-related dementia, Parkinson's disease, amyotrophic lateral sclerosis, retinitis pigmentosa, spinal muscular atrophy and cerebellar degeneration), AIDS, myelodysplastic syndromes, aplastic anemia, ischemic injury associated myocardial infarctions, stroke and reperfusion injury, arrhythmia, atherosclerosis, toxin-induced or alcohol induced liver diseases, hematological diseases (including, but not limited to, chronic anemia and aplastic anemia), degenerative diseases of the musculoskeletal system (including, but not limited to, osteoporosis and arthritis), aspirin-sensitive rhinosinusitis, cystic fibrosis, multiple sclerosis, kidney diseases, and cancer pain.

The compounds of this invention may be used in combination with known anti-cancer treatments such as radiation therapy or with cytostatic and cytotoxic agents including, but not limited to, microtuble-stabilizing agents, microtuble-disruptor agents, alkylating agents, anti-metabolites, epidophyllotoxin, an antineoplastic enzyme, a topoisomerase inhibitor, procarbazine, mitoxantrone, platinum coordination complexes, biological response modifiers, growth inhibitors, hormonal/anti-hormonal therapeutic agents, haematopoietic growth factors, and the like.

Classes of anti-cancer agents which may be used in combination with the formula I compounds of this invention include, but are not limited to, the anthracycline family of drugs, the vinca drugs, the mitomycins, the bleomycins, the cytotoxic nucleosides, the taxanes, the epothilones, discodermolide, the pteridine family of drugs, diynenes, aromatase inhibitors, and the podophyllotoxins. Particluar members of those classes include, for example, paclitaxel, docetaxel, 7-O-methylthiomethylpaclitaxel (disclosed in U.S. 5,646,176), 3'*-tert*-butyl-3'-N-*tert*-butyloxycarbonyl-4-deacetyl-3'-dephenyl-3'-N-debenzoyl-4-O-methoxycarbonylpaclitaxel (disclosed in WO 01/56565, C-4 methyl carbonate paclitaxel (disclosed in WO 94/14787), epothilone A, epothilone B, epothilone C, epothilone D, desoxyepothilone A, desoxyepothilone B, [1S-[1R*,3R*(E),7R*,10S*,11R*,12R*, 16S*]]-7,11-dihydroxy-8,8,10,12,16-pentamethyl-3-[1-methyl-2-(2-methyl-4-thiazolyl)ethenyl]-4-aza-17-oxabicyclo[14.1.0]heptadecane-5,9-dione (disclosed in WO 99/02514), [1S-[1R*,3R*(E),7R*,10S*,11R*,12R*,16S*]]-3-[2-[2-(aminomethyl)-4-thiazolyl]-1-methylethenyl]-7,11-dihydroxy-8,8,10,12,16-pentamethyl-4,17-dioxabicyclo[14.1.0]heptadecane-5,9-dione (disclosed in U.S. 6,262,094, doxorubicin, carminomycin, daunorubicin, aminopterin, methotrexate, methopterin, dichloro-methotrexate, mitomycin C, porfiromycin, 5-fluorouracil, 6-mercaptopurine, gemcitabine, cytosine arabinoside, podophyllotoxin or podophyllotoxin derivatives such as etoposide, etoposide phosphate or teniposide, melphalan, vinblastine, vincristine, leurosidine, vindesine, leurosine, and the like. Other useful anti-cancer agents which may be used in combination with the compounds of the present invention include, but are not limited to, estramustine, cisplatin, carboplatin, cyclophosphamide, bleomycin, tamoxifen, ifosamide, melphalan, hexamethyl melamine, thiotepa, cytarabin, idatrexate, trimetrexate, dacarbazine, L-asparaginase, camptothecin, CPT-11, topotecan, ara-C, bicalutamide, flutamide, leuprolide, pyridobenzoindole derivatives, interferons, interleukins, and the like. In addition, the compounds of this invention may be used in combination with inhibitors of farnesyl protein transferase such as those described in U.S. 6,011,029; anti-angiogenic agents such as angiostatin and endostatin; kinase inhibitors such as her2 specific antibodies; and modulators of p53 transactivation.

If formulated as a fixed dose, such combination products employ the compounds of this invention within the dosage range described below and the other pharmaceutically active agent within its approved dosage range. Compounds of formula I may be used sequentially, in any order, with known anti-cancer or cytotoxic agents when a combination formulation is inappropriate.

The present invention also provides pharmaceutical compositions which comprise a compound of this invention and a pharmaceutically acceptable carrier. It should be noted that, in the context of the pharmaceutical compositions of the present invention, the compounds of the invention, or compounds of formula I, refer to the free base, enantiomers, diastereomers as well as pharmaceutically acceptable salts. Examples of such pharmaceutically acceptable salts include, but are not limited to, hydrochloride, dihydrochloride, sulfate, trifluoroacetate, mixture of trifluoroacetate and hydrochloride, tartarate, fumarate, succinate, maleate, citrate, methanesulfonate, bromate and iodate salts. Also included are salts formed with other organic and inorganic acids such as hydroxymethane sulfonic acid, acetic acid, benzenesulfonic acid, toluenesulfonic acid and various others, e.g., nitrates, phosphates, borates, benzoates, ascorbates, salicylates, and the like. These salts include racemic forms as well as enantiomers and diastereomers (such as, for example, D-tartarate and L-tartarate salts). In addition, pharmaceutically acceptable salts of compounds of formula I may be formed with alkali metals such as sodium, potassium and lithium; alkaline earth metals such as calcium and magnesium; organic bases such as dicyclohexylamine, tributylamine, and pyridines, and the like; and amino acids such as arginine, lysine and the like.

The pharmaceutical compositions of the present invention may further comprise one or more pharmaceutically acceptable additional ingredient(s) such as alum, stabilizers, antimicrobial agents, buffers, coloring agents, flavoring agents, and the like. The compounds and compositions of this invention may be administered orally or parenterally including the intravenous, intramuscular, intraperitoneal, subcutaneous, rectal and topical routes of administration.

For oral use, the compounds and compositions of this invention may be administered, for example, in the form of tablets or capsules, or as solutions or suspensions. In the case of tablets for oral use, carriers which are commonly used include lactose and corn starch, and lubricating agents such as magnesium stearate are commonly added. For oral administration in capsule form, useful carriers include lactose and corn starch. When aqueous suspensions are used for oral administration, emulsifying and/or suspending agents are commonly added. In addition, sweetening and/or flavoring agents may be added to the oral compositions. For intramuscular, intraperitoneal, subcutaneous and intravenous use, sterile solutions of the active ingredient(s) are usually employed, and the pH of the solutions should be suitably adjusted and buffered. For intravenous use, the total concentration of the solute(s) should be controlled in order to render the preparation isotonic.

Daily dosages for human administration of the compounds of this invention will normally be determined by the prescribing physician with the dosages generally varying according to the age, weight, route of administration, and response of the individual patient, as well as the severity of the patient's symptoms. A formula I compound of this invention is preferably administered to humans in an amount from about 0.001 mg/kg of body weight to about 100 mg/kg of body weight per day, more preferably from about 0.01 mg/kg of body weight to about 50 mg/kg of body weight per day, and most preferably from about 0.1 mg/kg of body weight to about 20 mg/kg of body weight per day.

### cdc2/cyclin B1 Kinase Assay

cdc2/cyclin B1 kinase activity was determined by monitoring the incorporation of ³²P into histone HI. The reaction consisted of 50 ng baculovirus expressed GST-cdc2, 75 ng baculovirus expressed GST-cyclin B1, 1 µg histone HI (Boehringer Mannheim), 0.2 µCi of ³²P γ-ATP and 25 µM ATP in kinase buffer (50 mM Tris, pH 8.0, 10 mM MgCl₂, 1 mM EGTA, 0.5 mM DTT). The reaction was incubated at 30 °C for 30 minutes and then stopped by the addition of cold trichloroacetic acid (TCA) to a final concentration of 15 % and incubated on ice for 20 minutes. The reaction was harvested onto GF/C unifilter plates (Packard) using a Packard Filtermate Universal harvester, and the filters were counted on a Packard TopCount 96-well liquid scintillation counter (Marshak, D.R., Vanderberg, M.T., Bae, Y.S., Yu, I.J., *J*. *of Cellular Biochemistry,* 45, 391-400 (1991)).

### cdk2/cyclin E Kinase Assay

cdk2/cyclin E kinase activity was determined by monitoring the incorporation of ³²P into the retinoblastoma protein. The reaction consisted of 2.5 ng baculovirus expressed GST-cdk2/cyclin E, 500 ng bacterially produced GST-retinoblastoma protein (aa 776-928), 0.2 µCi ³²P γ-ATP and 25 µM ATP in kinase buffer (50 mM Hepes, pH 8.0, 10 mM MgCl₂, 5 mM EGTA, 2 mM DTT). The reaction was incubated at 30 °C for 30 minutes and then stopped by the addition of cold trichloroacetic acid (TCA) to a final concentration of 15 % and incubated on ice for 20 minutes. The reaction was harvested onto GF/C unifilter plates (Packard) using a Packard Filtermate Universal harvester, and the filters were counted on a Packard TopCount 96-well liquid scintillation counter.

### cdk 4/cyclin D1 Kinase Activity

cdk4/cyclin D 1 kinase activity was determined by monitoring the incorporation of ³²P in to the retinoblastoma protein. The reaction consisted of 165 ng baculovirus expressed as GST-cdk4, 282 ng bacterially expressed as S-tag cyclin D1, 500 ng bacterially produced GST-retinoblastoma protein (aa 776-928), 0.2 µCi ³²P γ-ATP and 25 µM ATP in kinase buffer (50 mM Hepes, pH 8.0, 10 mM MgCl₂, 5 mM EGTA, 2 mM DTT). The reaction was incubated at 30 °C for 1 hour and then stopped by the addition of cold trichloroacetic acid (TCA) to a final concentration of 15 % and incubated on ice for 20 minutes. The reaction was harvested onto GF/C unifilter plates (Packard) using a Packard Filtermate Universal harvester, and the filters were counted on a Packard TopCount 96-well liquid scintillation counter (Coleman, K.G., Wautlet, B.S., Morissey, D, Mulheron, J.G., Sedman, S., Brinkley, P., Price, S., Webster, K.R. (1997) Identification of CDK4 Sequences involved in cyclin D, and p16 binding. *J*. *Biol*. *Chem*. 272,30:18869-18874).

In order to facilitate a further understanding of the invention, the following examples are presented primarily for the purpose of illustrating more specific details thereof. The scope of the invention should not be deemed limited by the examples, but encompasses the entire subject matter defined in the claims.

### Example 1

### Preparation of N-[5-[[[5-(1,1-Dimethylethyl)-2-oxazolyl]methyl]thio]-2-thiazolyl]-4-piperidinecarboxamide

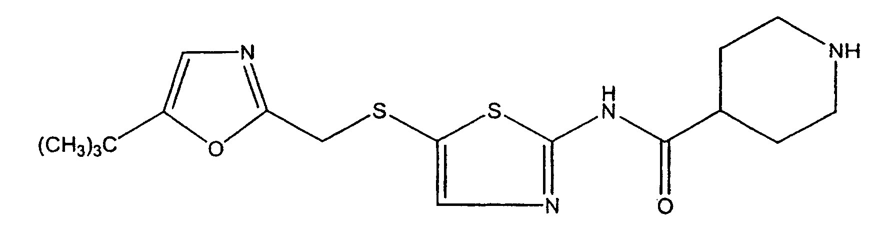

### A. N-(3,3-Dimethyl-2-butanonyl)hexamethylenetetraminium bromide

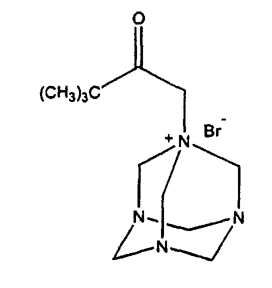

1-Bromopinacolone (179.05 g, 1 mol, I eq) was combined in 2 L of acetone with hexamethylenetetramine (154.21 g, 1.1 mol, 1.1 eq). The reaction mixture was stirred under N₂ at rt for 26 h. The resulting slurry was filtered. The filter cake was washed with ether (3 x 50 mL) and dried *in vacuo* at 50 °C overnight to provide 330 g (100 %) of N-(3,3-dimethyl-2-butanonyl)hexamethylenetetraminium bromide containing 7 % hexamethylenetetramine. HPLC R.T.=0.17 min (Phenomenex 5 µm C 18 column 4.6 x 50 mm, 10-90 % aqueous methanol over 4 minutes containing 0.2 % phosphoric acid, 4 mL/min, monitoring at 220 nm).

### B. 1-Amino-3,3-dimethyl-2-butanone hydrochloride

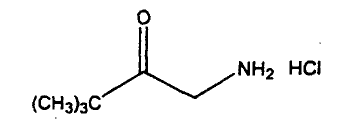

N-(3,3-Dimethyl-2-butanonyl)hexamethylenetetraminium bromide (400 g, 1.254 mol, 1 eq) was combined in 2 L of ethanol with 12 N aqueous HCl (439 mL, 5.26 mol, 4.2 eq). The reaction mixture was stirred at 75 °C for 1 h and then allowed to cool to rt. The resulting slurry was filtered. The filtrate was concentrated *in vacuo* and isopropyl alcohol was added. The solution was filtered again. Addition of 1.2 L of ether caused the desired material to precipitate from solution. The material was filtered, washed with ether (2 x 300 mL), and dried *in vacuo* at 50 °C overnight to provide 184.1 g (97 %) of 1-amino-3,3-dimethyl-2-butanone hydrochloride.

### C. N-Chloroacetyl-1-amino-3,3-dimethyl-2-butanone

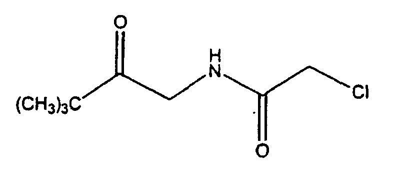

1-Amino-3,3-dimethyl-2-butanone hydrochloride (130.96 g, 0.8637 mol, 1 eq) was dissolved in 3.025 L of CH₂Cl₂ under N₂ at -5 °C. Triethylamine (301 mL, 2.16 mol, 2.5 eq) was added followed by chloroacetyl chloride (75.7 mL, 0.450 mol, 1.1 eq) in 175 mL of CH₂Cl₂. The resulting slurry was stirred at -5 to -10 °C for 2 h. Water (1.575 L) was added followed by 175 mL of concentrated HCl. The organic phase was washed a second time with 1.75 L of 10 % aqueous HCl, and then with 500 mL of water. The organic phase was dried over Na₂SO₄ and concentrated *in vacuo* to provide 155.26 g (93.8 %) of N-chloroacetyl-1-amino-3,3-dimethyl-2-butanone HPLC R.T.=2.27 min (Phenomenex 5 µm C 18 column 4.6 x 50 mm, 10-90 % aqueous methanol over 4 minutes containing 0.2 % phosphoric acid, 4 mL/min, monitoring at 220 nm).

### D. 5-t-Butyl-2-chloromethyloxazole

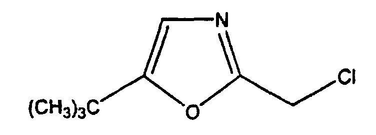

N-Chloroacetyl-1-amino-3,3-dimethyl-2-butanone (180.13 g, 0.9398 mol, 1 eq) was combined with phosphorus oxychloride (262 mL, 2.8109 mol, 3 eq) under N₂. The reaction mixture was heated at 105 °C for 1 h. The mixture was cooled to rt and then quenched with 1.3 kg of ice. The aqueous phase was extracted with ethyl acetate (1L, then 2 x 500 mL). The organic extracts were washed with saturated aqueous NaHCO₃ (4 x 1 L) which was back-extracted several times with ethyl acetate. The organic phases were combined, washed with saturated aqueous NaHCO₃ (500 mL) followed by saturated aqueous NaCl (300 mL), dried over MgSO₄, and concentrated *in vacuo* to give a brown oil. The crude material was distilled under high vacuum at 100 °C to provide 155.92 g (96 %) of 5-*t*-butyl-2-chloromethyloxazole. HPLC R.T.=3.62 min (Phenomenex 5 µm C18 column 4.6 x 50 mm, 10-90 % aqueous methanol over 4 minutes containing 0.2 % phosphoric acid, 4 mL/min, monitoring at 220 nm).

### Alternate method using Burgess' reagent:

As an alternative, 5-*t*-butyl-2-chloromethyloxazole may be prepared by reaction of a suitable chloroamide with 2 eq of Burgess' salt in tetrahydrofuran.

### E. 5-t-Butyl-2-(5-thioureamethyl)oxazole

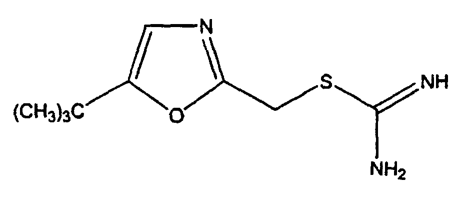

5-*t*-Butyl-2-chloromethyloxazole (1.77 g, 10.2 mmol, 1.02 eq) was combined with thiourea (0.76 g, 9.98 mmol, 1 eq) under N₂ in 10 mL of absolute ethanol. The reaction mixture was heated at reflux for 1.5 h. The mixture was cooled to rt and concentrated *in vacuo*. Trituration of the resulting crude material with *t*-butyl methyl ether provided 2.32 g (93 %) of 5-*t*-butyl-2-(5-thioureamethyl)oxazole. HPLC R.T.=2.05 min (Phenomenex 5 µm C 18 column 4.6 x 50 mm, 10-90 % aqueous methanol over 4 minutes containing 0.2 % phosphoric acid, 4 mL/min, monitoring at 220 nm); ¹H NMR (*d*-DMSO): δ 9.48 (s, 3H), 6.85 (s, 1H), 4.73 (s, 2H), 1.24 (s, 9H).

### F. 2-Amino-5-[[[5-(1,1-dimethylethyl)-2-oxazolyl]methyl]thio]thiazole

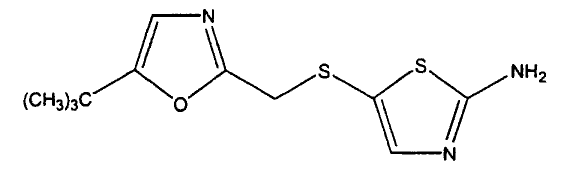

5-*t*-Butyl-2-(5-thioureamethyl)oxazole (1.25 g, 5 mmol, 1 eq) was added to a mixture of NaOH (3.0 g, 75 mmol, 15 eq), water (10 mL), toluene (10 mL), and tetrabutylammonium sulfate (50 mg, 0.086 mmol, 0.017 eq). 5-Bromo-2-aminothiazole hydrobromide (1.70 g, 5 mmol, 1 eq) was added and the reaction mixture was stirred at rt for 14.5 h. The mixture was diluted with water and extracted twice with ethyl acetate. The organic extracts were washed with water (4 x 10 mL), dried over MgSO₄, and concentrated *in vacuo* to provide 1.1 g (82 %) of 2-amino-5-[[[5-(1,1-dimethylethyl)-2-oxazolyl]methyl]thio]thiazole. HPLC 86.3 % at 2.75 min (Phenomenex 5 µm C 18 column 4.6 x 50 mm, 10-90 % aqueous methanol over 4 minutes containing 0.2 % phosphoric acid, 4 mL/min, monitoring at 220 nm); ¹H NMR (CDCl₃): δ 6.97 (s, 1H), 6.59 (s, 1H), 5.40 (br s, 2H), 3.89 (s, 2H), 1.27 (s, 9H).

### G. N-[5-[[[5-(1,1-Dimethylethyl)-2-oxazolyl]methyl]thio]-2-thiazolyl]-4-piperidinecarboxamide

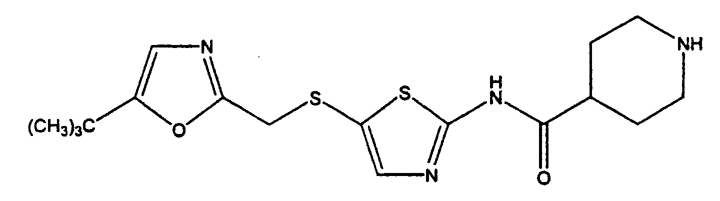

1-(3-Dimethylaminopropyl)-3-ethylcarbodiimide hydrochloride (13.8 g, 72 mmol, 2 eq) was added to a mixture of 2-amino-5-[[[5-(1,1-dimethylethyl)-2-oxazolyl]methyl]thio]thiazole (9.6 g, 35.6 mmol, I eq), N-*t*-butoxycarbonylisonipecotic acid (12.6 g, 55 mmol, 1.5 eq), 4-(dimethylamino)pyridine (2 g, 16 mmol, 0.45 eq), N,N-dimethylformamide (36 mL) and CH₂Cl₂ (100 mL). The clear reaction mixture became cloudy as it was stirred at rt for 3.5 h. Water (300 mL) and ethyl acetate (200 mL) were added and the resulting precipitate was filtered off. The filtrate was extracted with ethyl acetate. The organic extracts were dried over MgSO₄ and concentrated *in vacuo* to provide a yellow solid which was combined with the precipitate. The solid was boiled in a mixture of ethanol, acetone, and water for 20 min. The solid was filtered, washed with an ethanol/water mixture, and dried to give a BOC-coupled intermediate (16.6 g) as a white solid. A magnetically-stirred suspension of the BOC-coupled intermediate (20.2 g) in 200 mL of chloroform was warmed until homogeneous then a solution of 4N HCl in dioxane (31 mL) was added at 55°C. Gas was evolved and a precipitate formed within a few minutes. After 7 hr, HPLC indicated the reaction was about 2/3 complete. Additional 4N HCl in dioxane (10 mL) was introduced and the reaction mixture was stirred at 60 °C for 1 hr followed by room temperature overnight. A third portion (10 mL) of 4N HCl in dioxane was added and the reaction mixture stirred at 45 °C for 6 hr. The resultant heavy suspension was stirred at room temperature overnight then cooled in an ice-bath and saturated aq sodium bicarbonate solution (200 mL) was added. Gas was evolved during the addition. The heavy suspension became homogeneous then formed a light suspension. The light suspension was treated with 6 g of solid sodium carbonate, heated at 60 °C for 20 minutes, and diluted with chloroform (100 mL). The aqueous phase was separated and extracted with chloroform (2 x 100 mL). The organics were combined, washed with brine (100 mL), dried (sodium carbonate and sodium sulfate), and concentrated *in vacuo* to give a yellow solid. The yellow solid was dissolved in warm 95 % ethanol (200 mL), diluted with water (200 mL), warmed until homogeneous, and cooled overnight in a refrigerator. The resultant solid was collected, washed with 1:1 ethanol/water, and dried at 50 °C under vacuum overnight to give N-[5-[[[5-(1,1-dimethylethyl)-2-oxazolyl]methyl]thio]-2-thiazolyl]-4-piperidinecarboxamide as a white solid (13.3 g, mp 171-173 °C). LC/MS: 381 [M+H]⁺; HPLC: HI >99 % at 3.12 min (YMC S5 ODS column 4.6 x 50 mm, 10-90 % aqueous methanol over 4 minutes containing 0.2 % phosphoric acid, 4 mL/min, monitoring at 220 nm).

### Example 2

### Preparation of (±)-N-[5-[[[5-(1,1-Dimethylethyl)-2-oxazolyl]methyl]thio]-2-thiazolyl]-3-piperidinecarboxamide

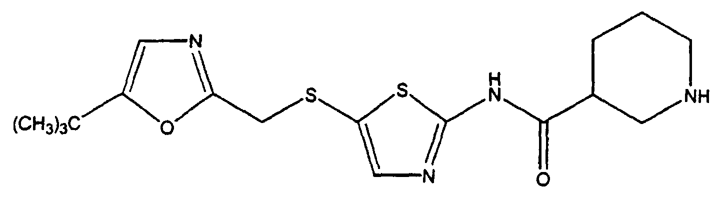

### A. (±)-N-t-butoxycarbonyl-nipecotic acid

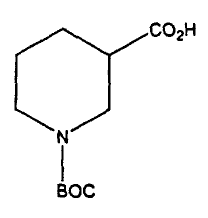

Nipecotic acid (1.3 g, 10 mmol, 1 eq) was combined with 10 mL of dioxane, 2 mL of acetonitrile, 10 mL of water, and 10 mL of 1N aqueous NaOH (1 eq). Di-*t*-butyl dicarbonate (3.3 g, 15 mmol, 1.5 eq) was added and the reaction mixture was stirred at rt overnight. The reaction mixture was concentrated *in vacuo* to remove organic solvent and 10 % aqueous citric acid was added The mixture was extracted with ethyl acetate (3 x 100 mL). The organic extracts were dried over Na₂SO₄, filtered through silica gel, and concentrated *in vacuo*. The crude material was recrystallized from ethyl acetate and hexanes to provide 2.2 g (96 %) of (±)-N-*t*-butoxycarbonyl-nipecotic acid as a white solid.

### B. (±)-N-[5-[[[5-(1,1-Dimethylethyl)-2-oxazolyl]methyl]thio]-2-thiazolyl]-(N-t-butoxycarbonyl)-3-piperidinecarboxamide

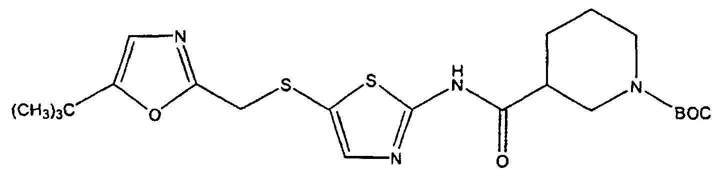

1-(3-Dimethylaminopropyl)-3-ethylcarbodiimide hydrochloride (383 mg, 2 mmol, 2 eq) was added to a mixture of 2-amino-5-[[[5-(1,1-dimethylethyl)-2-oxazolyl]methyl]thio]thiazole (270 mg, 1 mmol, 1 eq), N-*t*-butoxycarbonyl-nipecotic acid (344 mg, 1.5 mmol, 1.5 eq), 4-(dimethylamino)pyridine (61 mg, 0.5 mmol, 0.5 eq), N,N-dimethylformamide (1 mL) and CH₂Cl₂ (6 mL). The reaction mixture was stirred at rt for 1.3 h. Triethylamine (0.28 mL, 2 mmol, 2 eq) was added, and the reaction mixture was stirred for 1h. Additional N-*t*-butoxycarbonyl-nipecotic acid (340 mg), triethylamine (0.28 mL) and 1-(3-dimethylaminopropyl)-3-ethylcarbodiimide hydrochloride (380 mg) were added. After 1 h, no further change was observed. Additional 4-(dimethylamino)pyridine, N,N-dimethylformamide, triethylamine and starting acid were added and the reaction was stirred overnight at rt. The resulting black solution was diluted with saturated aqueous NaHCO₃ and extracted with CH₂Cl₂. The organic extracts were dried, concentrated *in vacuo*, and purified by flash chromatography on silica gel eluting with a gradient of 50-100 % ethyl acetate in hexanes to provide 397 mg (83 %) of (±)-N-[5-[[[5-(1,1-dimethylethyl)-2-oxazolyl]methyl]thio]-2-thiazolyl]-(N-*t*-butoxycarbonyl)-3-piperidinecarboxamide as a yellow glassy solid.

### C. (±)-N-[5-[[[5-(1,1-Dimethylethyl)-2-oxazolyl]methyl]thio]-2-thiazolyl]-3-piperidinecarboxamide

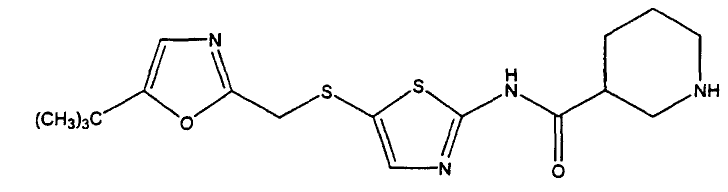

(±)-N-[5-[[[5-(1,1-Dimethylethyl)-2-oxazolyl]methyl]thio]-2-thiazolyl]-(N-*t*-butoxycarbonyl)-3-piperidinecarboxamide (355 mg, 0.74 mmol, 1 eq) was dissolved in 3 mL of CH₂Cl₂. Trifluoroacetic acid (3 mL) was added, and the mixture was stirred at rt for 20 min. The reaction mixture was concentrated *in vacuo* and neutralized with saturated aqueous NaHCO₃. The resulting mixture was extracted with ethyl acetate. The organic extracts were dried over Na₂SO₄, concentrated *in vacuo*, and recrystallized from ethyl acetate to provide 142 mg (50 %) of (±)-N-[5-[[[5-(1,1-dimethylethyl)-2-oxazolyl]methyl]thio]-2-thiazolyl]-3-piperidinecarboxamide as a white solid. MS: 381 [M+H]⁺; HPLC: 100 % at 3.15 min (YMC S5 ODS column 4.6 x 50 mm, 10-90 % aqueous methanol over 4 minutes containing 0.2 % phosphoric acid, 4 mL/min, monitoring at 220 nm).

### Example 3

### Preparation of (±)-1-(2,3-Dihydroxypropyl)-N-[5-[[[5-(1,1-dimethylethyl)-2-oxazolyl]methyl]thio]-2-thiazolyl]-4-piperidinecarboxamide

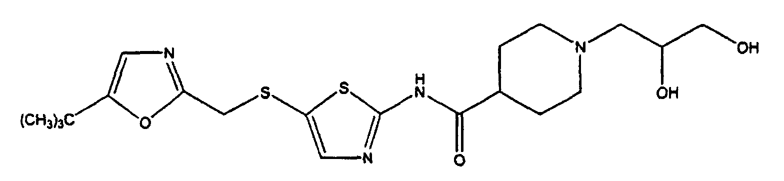

N-[5-[[[5-( 1,1-Dimethylethyl)-2-oxazolyl]methyl]thio]-2-thiazolyl]-4-piperidinecarboxamide (66 mg, 0.17 mmol, 1 eq) was combined with glyceraldehyde (69 mg, 0.77 mmol, 4.5 eq), sodium triacetoxyborohydride (163 mg, 0.77 mmol, 4.5 eq) and 1,2-dichloroethane (4 mL). The resulting suspension was stirred at rt for 4 h. Methanol (1 mL) was added and the reaction mixture was stirred at rt overnight, concentrated *in vacuo* and purified by preparative HPLC to provide 69 mg (59 %) of (±)-1-(2,3-dihydroxypropyl)-N-[5-[[[5-(1,1-dimethylethyl)-2-oxazolyl]methyl]thio]-2-thiazolyl]-4-piperidinecarboxamide as a white solid. MS: 455 [M+H]⁺; HPLC: 100 % at 3.06 min (YMC S5 ODS column 4.6 x 50 mm, 10-90 % aqueous methanol over 4 minutes containing 0.2 % phosphoric acid, 4 mL/min, monitoring at 220 nm).

### Example 4

### Preparation of N-[5-[[[5-(1,1-Dimethylethyl)-2-oxazolyl]methyl]thio]-2-thiazolyl]-1-(1-methylethyl)-4-piperidinecarboxamide

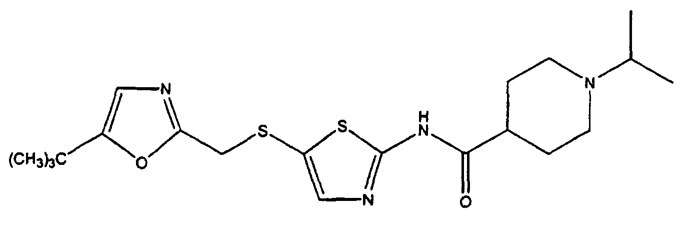

### A. Ethyl 1-(1-methylethyl)-4-piperidine carboxylate

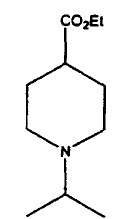

Ethyl isonipecotate (3.2 g, 20 mmol, 1 eq) was combined with acetone (5.8 g, 100 mmol, 5 eq), sodium triacetoxyborohydride (10.5 g, 50 mmol, 2.5 eq) and 1,2-dichloroethane (200 mL). The reaction mixture was stirred at rt for 72 h. Saturated aqueous NaHCO₃ was added, and the mixture was extracted with CH₂Cl₂. The organic extracts were dried, filtered through a silica gel pad, and concentrated *in vacuo* to provide 3.72 g (93 %) of ethyl 1-(1-methylethyl)-4-piperidine carboxylate as a colorless liquid.

### B. 1-(1-Methylethyl)-4-piperidine carboxylic acid

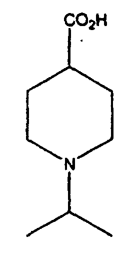

Ethyl 1-(1-methylethyl)-4-piperidine carboxylate (3.6 g, 18 mmol, 1 eq) was combined with barium hydroxide octahydrate (10.4 g, 33 mmol, 1.8 eq) in a mixture of 70 mL of water with 44 mL of ethanol. The mixture was heated at 60 °C for 1.3 h. The reaction mixture was concentrated *in vacuo* and diluted with 70 mL of water. Ammonium carbonate (6.9 g, 87 mmol, 4.8 eq) was added portionwise and the reaction mixture was stirred at rt overnight. The mixture was filtered through diatomaceous earth, concentrated, and lyophilized to provide 3.1 g (100 %) of 1-(1-methylethyl)-4-piperidine carboxylic acid as a white solid.

### C. N-[5-[[[5-(1,1-Dimethylethyl)-2-oxazolyl]methyl]thio]-2-thiazolyl]-1-(1-methylethyl)-4-piperidinecarboxamide

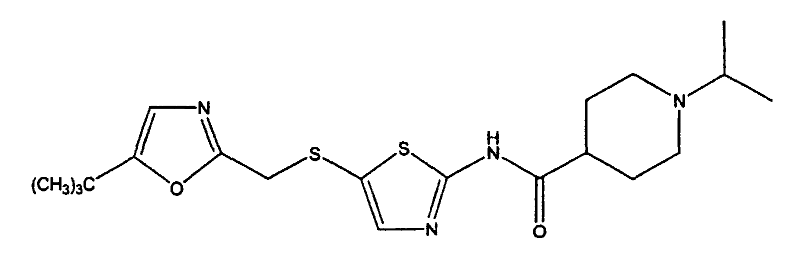

1-(3-Dimethylaminopropyl)-3-ethylcarbodiimide hydrochloride (1.0 g, 5.2 mmol, 2 eq) was added to a mixture of 2-amino-5-[[[5-(1,1-dimethylethyl)-2-oxazolyl]methyl]thio]thiazole (0.7 g, 2.6 mmol, 1 eq), 1-(1-methylethyl)-4-piperidine carboxylic acid (0.78 g, 3.9 mmol, 1.5 eq), 4-(dimethylamino)pyridine (0.16 g, 1.3 mmol, 0.5 eq), N,N-dimethylformamide (2.6 mL) and CH₂Cl₂ (7.8 mL). The reaction mixture was stirred at rt for 1 h, diluted with 30 mL of water and extracted with ethyl acetate (2 x 70 mL). The organic extracts were dried over Na₂SO₄, concentrated in *vacuo*, and purified by flash chromatography on silica gel eluting with a gradient of 5-10 % triethylamine in ethyl acetate. The material was recrystallized from ethanol and water to provide 0.93 g (85 %) of N-[5-[[[5-(1,1-dimethylethyl)-2-oxazolyl]-methyl]thio]-2-thiazolyl]-1-(1-methylethyl)-4-piperidinecarboxamide as a yellowish solid. MS: 423 [M+H]⁺; HPLC: 100 % at 3.15 min (YMC S5 ODS column 4.6 x 50 mm, 10-90 % aqueous methanol over 4 minutes containing 0.2 % phosphoric acid, 4 mL/min, monitoring at 220 nm).

### Example 5

### Preparation of 1-Cyclopropyl-N-[5-[[[5-(1,1-dimethylethyl)-2-oxazolyl]methyl]thio]-2-thiazolyl]-4-piperidinecarboxamide

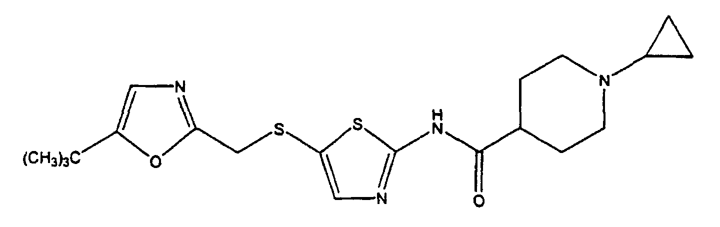

### A. 1-Cyclopropyl-4-piperidine carboxylic acid

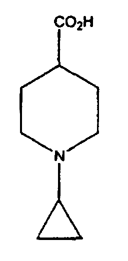

Ethyl isonipecotate (1.57 g, 10 mmol, 1 eq) was combined with ((1-ethoxycyclopropyl)oxy)trimethyl silane (8.7 g, 50 mmol, 5 eq) in 100 mL of methanol. Acetic acid (5.7 mL, 100 mmol, 10 eq) and molecular sieves were added. After 30 min at rt, sodium triacetoxyborohydride (2.5 g, 40 mmol, 4 eq) was added and the reaction mixture was heated at 65°C overnight. The reaction mixture was cooled and Na₂CO₃ (20 g) was added. The mixture was stirred at rt for 2 h and filtered through diatomaceous earth. The diatomaceous earth was washed with methanol. The filtrates were combined, concentrated *in vacuo*, diluted with water, and extracted with ethyl acetate. The organic extracts were dried, filtered through a silica gel pad, and concentrated *in vacuo* to provide 2.4 g of colorless liquid. This material was combined with barium hydroxide octahydrate (5.7 g, 18 mmol, 1.8 eq) in a mixture of 38 mL of water with 24 mL of ethanol. The mixture was heated at 60 °C for 1 h. The reaction mixture was concentrated *in vacuo* and diluted with 38 mL of water. Ammonium carbonate (3.8 g) was added portionwise and the reaction was stirred at rt for 2 h. The mixture was filtered through diatomaceous earth, washing with water. The filtrate was washed with ethyl acetate. Concentration of the aqueous phase provided 1.56 g (92 %) of 1-cyclopropyl-4-piperidine carboxylic acid as a hygroscopic white solid.

### B. 1-Cyclopropyl-N-[5-[[[5-(1,1-dimethylethyl)-2-oxazolyl]-methyl]thio]-2-thiazolyl]-4-piperidinecarboxamide

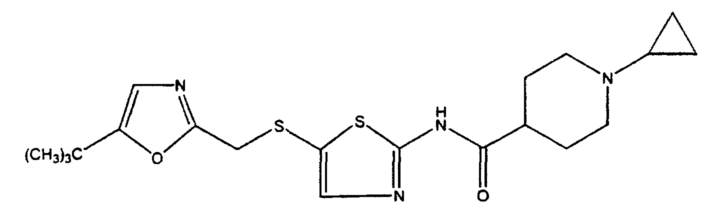

1-(3-Dimethylaminopropyl)-3-ethylcarbodiimide hydrochloride (1.0 g, 5.2 mmol, 2 eq) was added to a mixture of 2-amino-5-[[[5-(1,1-dimethylethyl)-2-oxazolyl]methyl]thio]thiazole (0.7 g, 2.6 mmol, 1 eq), 1-cyclopropyl-4-piperidine carboxylic acid (0.77 g, 3.9 mmol, 1.5 eq), 4-(dimethylamino)pyridine (0.16 g, 1.3 mmol, 0.5 eq), N,N-dimethylformamide (2.6 mL) and CH₂Cl₂ (7.8 mL). The reaction mixture was stirred at rt for 1 h, diluted with water (30 mL), and extracted with ethyl acetate (2 x 70 mL). The combined organic extracts were dried over anhydrous sodium sulfate, concentrated *in vacuo*, and purified by flash chromatography on silica gel eluting with a gradient of 0-10 % triethylamine in ethyl acetate. The material was crystallized from ethyl acetate and hexanes to provide 0.7 g (65 %) of 1-cyclopropyl-N-[5-[[[5-(1,1-dimethylethyl)-2-oxazolyl]methyl]thio]-2-thiazolyl]-4-piperidine-carboxamide as white crystals. MS: 421 [M+H]⁺; HPLC: 100 % at 3.13 min (YMC S5 ODS column 4.6 x 50 mm, 10-90 % aqueous methanol over 4 minutes containing 0.2 % phosphoric acid, 4 mL/min, monitoring at 220 nm).

### Example 6

### Preparation of N-[5-[[[5-(1,1-Dimethylethyl)-2-oxazolyl]methyl]thio]-2-thiazolyl]-1-(2-hydroxyethyl)-4-piperidinecarboxamide

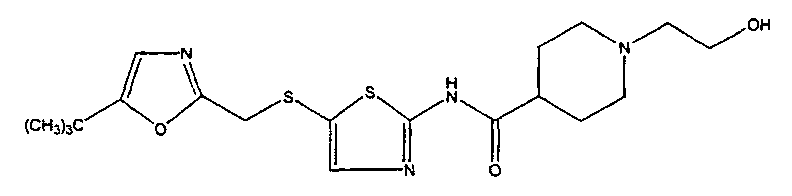

### A. N-[5-[[[5-(1,1-Dimethylethyl)-2-oxazolyl]methyl]thio]-2-thiazolyl]-1-(2-dimethyl-t-butylsilyloxyethyl)-4-piperidinecarboxamide

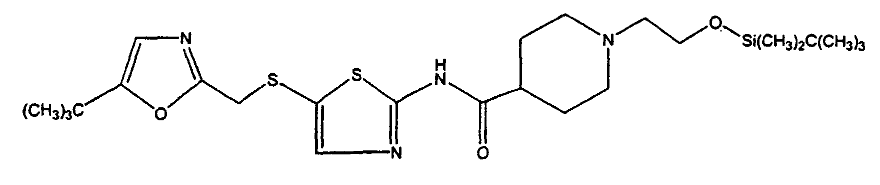

N-[5-[[[5-(1,1-dimethylethyl)-2-oxazolyl]methyl]thio]-2-thiazolyl]-4-piperidinecarboxamide (1.4 g, 3.68 mmol, 1 eq) was dissolved in 30 mL of N,N-dimethylformamide and 100 mL of tetrahydrofuran. 2-(Bromoethoxy)-*t*-butyldimethylsilane (0.79 mL, 3.68 mmol, 1 eq), and NaHCO₃ were added and the reaction mixture was stirred at 50 °C for 23 h. Additional 2-(bromoethoxy)-*t*-butyldimethylsilane (0.9 mL) was added, and the reaction mixture was stirred at 50 °C for 22 h, cooled, concentrated *in vacuo* and diluted with water (25 mL). The resultant aqueous mixture was extracted with ethyl acetate (50 mL). The organic extract was dried over Na₂SO₄, concentrated *in vacuo*, and purified by flash chromatography on silica gel eluting with a gradient of 0-5 % triethylamine in ethyl acetate to provide 1.7g (84 %) of N-[5-[[[5-(1,1-dimethylethyl)-2-oxazolyl]methyl]thio]-2-thiazolyl]-1-(2-dimethyl-*t*-butylsilyloxyethyl)-4-piperidinecarboxamide as a yellow solid. MS: 539 [M+H]⁺; HPLC: 98 % at 4.01 min (YMC S5 ODS column 4.6 x 50 mm, 10-90 % aqueous methanol over 4 minutes containing 0.2 % phosphoric acid, 4 mL/min, monitoring at 220 nm).

### B. N-[5-[[[5-(1,1-Dimethylethyl)-2-oxazolyl]methyl]thio]-2-thiazolyl]-1-(2-hydroxyethyl)-4-piperidinecarboxamide

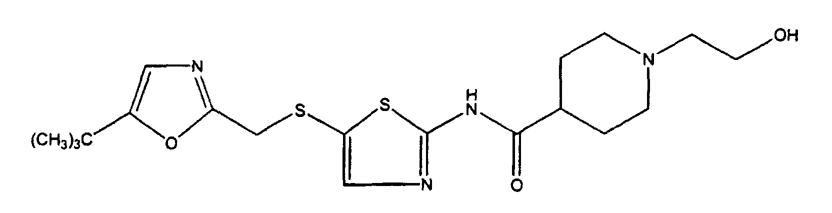

N-[5-[[[5-(1,1-Dimethylethyl)-2-oxazolyl]methyl]thio]-2-thiazolyl]-1-(2-dimethyl-*t*-butylsilyloxyethyl)-4-piperidinecarboxamide (1.45 g, 2.7 mmol, 1 eq) was dissolved in 100 mL of acetonitrile and combined with aqueous HF (48 % aqueous, 2.5 mL). The reaction mixture was stirred for 4 h at rt. An additional 2.5 mL of aqueous HF was added, and the reaction mixture was stirred overnight. Ethyl acetate (100 mL) and saturated aqueous NaHCO₃ (50 mL) were added. Additional solid NaHCO₃ was added to make the mixture basic. The mixture was extracted with ethyl acetate (2 x 50 mL). The organic extracts were dried over Na₂SO₄, filtered through a pad of silica gel, and concentrated *in vacuo*. The resulting white solid was crystallized from ethanol and water to provide 1.6 g (59 %) of N-[5-[[[5-(1,1-dimethylethyl)-2-oxazolyl]methyl]thio]-2-thiazolyl]-1-(2-hydroxyethyl)-4-piperidinecarboxamide as a white solid. MS: 425 [M+H]⁺; HPLC: 100 % at 3.05 min (YMC S5 ODS column 4.6 x 50 mm, 10-90 % aqueous methanol over 4 minutes containing 0.2 % phosphoric acid, 4 mL/min, monitoring at 220 nm).

### Example 7

### Preparation of (R)-N-[5-[[[5-(1,1-Dimethylethyl)-2-oxazolyl]methyl]thio]-2-thiazolyl]-3-piperidinecarboxamide hydrochloride

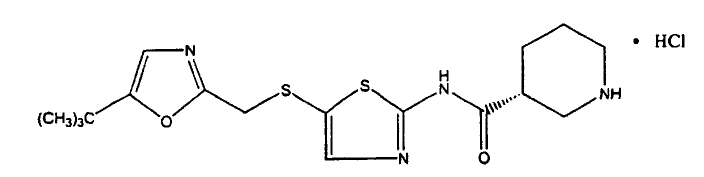

### A. (R)- and (S)-N-[5-[[[5-(1,1-Dimethylethyl)-2-oxazolyl]methyl]thio]-2-thiazolyl-(N-t-butoxycarbonyl)-3-piperidinecarboxamide

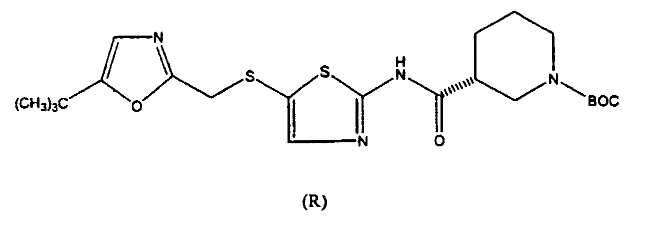

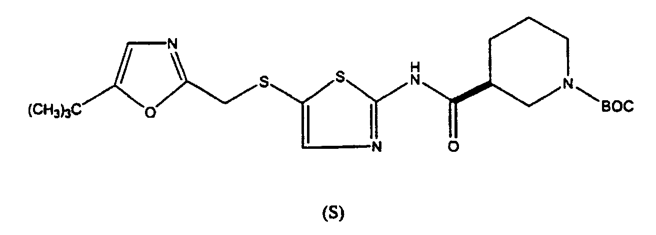

1-(3-Dimethylaminopropyl)-3-ethylcarbodiimide hydrochloride (3.8 g, 20 mmol, 2 eq) was added to a mixture of 2-amino-5-[[[5-(1,1-dimethylethyl)-2-oxazolyl]methyl]thio]thiazole (2.7 g, 10 mmol, 1 eq), N-*t*-butoxycarbonyl-nipecotic acid (3.4 g, 1.5 mmol, 1.5 eq), N,N-dimethylformamide (10 mL) and CH₂Cl₂ (30 mL). The reaction mixture was stirred at rt for 4 h. The resulting black solution was concentrated *in vacuo*, diluted with water (90 mL) and extracted with ethyl acetate (100 mL, then 2 x 75 mL). The organic extracts were dried over Na₂CO₃, concentrated *in vacuo*, and purified by flash chromatography on silica gel eluting with a gradient of 50-100 % ethyl acetate in hexanes to provide 3.8 g (79 %) of a yellow solid. The enantiomers were separated by chiral HPLC (Chiral Pak AD 5 x 50 cm 20 µ: eluent 10 % (0.1 % triethylamine in isopropanol) in hexanes; 45 mL/min, detection at 254 nm, loading 300 mg in 5 mL of isopropanol) to give each of the two optically pure isomers: 1.65 g of the R isomer and 1.65 g of the S isomer.

### B. (R)-N-[5-[[[5-(1,1-Dimethylethyl)-2-oxazolyl]methyl]thio]-2-thiazolyl]-3-piperidinecarboxamide hydrochloride

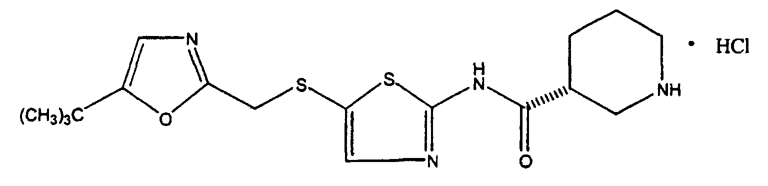

The (R) isomer of Part A (1.65 g, 3.43 mmol, 1 eq) was dissolved in 10 mL of CH₂Cl₂. Trifluoroacetic acid (6 mL) was added, and the mixture was stirred at rt for several hours. The reaction mixture was concentrated *in vacuo* and neutralized with saturated aqueous NaHCO₃. The resulting mixture was stirred with ethyl acetate for 1 h. The organic extracts were dried over Na₂SO₄ and concentrated *in vacuo* to provide a yellowish solid. The solid was dissolved in methanol and 1 eq of 1N aqueous HCl was added. The resulting solution was lyophilized to provide 1 g (77 %) of (R)-N-[5-[[[5-(1,1-dimethylethyl)-2-oxazolyl]methyl]thio]-2-thiazolyl]-3-pipendinecarboxamide hydrochloride as a yellow solid. MS: 381 [M+H]⁺; HPLC: 100 % at 3.14 min (YMC S5 ODS column 4.6 x 50 mm, 10-90 % aqueous methanol over 4 minutes containing 0.2 % phosphoric acid, 4 mL/min, monitoring at 220 nm).

### Example 8

### Preparation of (S)-N-[5-[[[5-(1,1-Dimethylethyl)-2-oxazolyl]methyl]thio]-2-thiazolyl]-3-piperidinecarboxamide hydrochloride

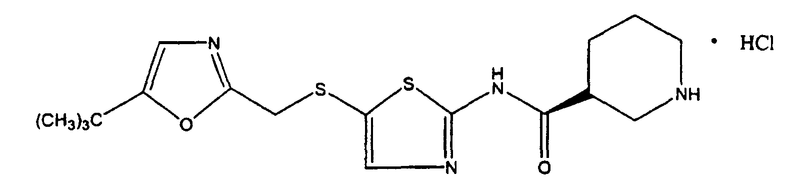

The (S) isomer of Example 7, Part A (1.65 g, 3.43 mmol, 1 eq) was dissolved in 10 mL of CH₂Cl₂. Trifluoroacetic acid (6 mL) was added, and the mixture was stirred at rt for several hours. The reaction was concentrated in *vacuo* and neutralized with saturated aqueous NaHCO₃. The resulting mixture was stirred with ethyl acetate for 1 h. The organic extracts were dried over Na₂SO₄ and concentrated *in vacuo* to provide a yellowish solid. The solid was dissolved in methanol and 1 eq of 1N aqueous HCl was added. The resulting solution was lyophilized to provide 0.918 g (70 %) of (S)-N-[5-[[[5-(1,1-dimethylethyl)-2-oxazolyl]methyl]thio]-2-thiazolyl]-3-piperidinecarboxamide hydrochloride as a yellow solid. MS: 381 [M+H]⁺; HPLC: 100 % at 3.15 min (YMC S5 ODS column 4.6 x 50 mm, 10-90 % aqueous methanol over 4 minutes containing 0.2 % phosphoric acid, 4 mL/min, monitoring at 220 nm).

### Example 9

### Preparation of cis-4-Amino-N-[5-[[[5-(1,1-dimethylethyl)-2-oxazolyl]-methyl]thio]-2-thiazolyl]cyclohexylcarboxamide hydrochloride and trans-4-Amino-N-[5-[[[5-(1,1-dimethylethyl)-2-oxazolyl]methyl]thio]-2-thiazolyl]-cyclohexylcarboxamide hydrochloride

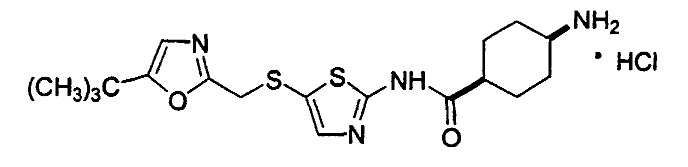

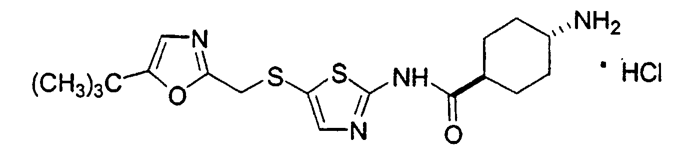

### A. 4-(t-Butoxycarbonylamino)cyclohexane carboxylic acid

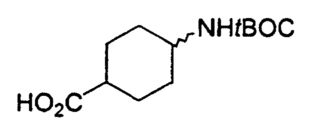

To a solution of 2.86 g (20 mmol) of 4-aminocyclohexane carboxylic acid in 40 mL of 0.5M aqueous NaOH solution, 20 mL of dioxane and 4 mL of acetonitrile was added a total of 6.5 g (30 mmol) of tBoc anhydride at room temperature. After 20 h, 100 mL of ethyl acetate and 100 mL of 10 % aqueous citric acid solution were introduced. The aqueous layer which formed was separated and extracted with three-50 mL portions of ethyl acetate. The organic phases were combined, dried (sodium sulfate) and concentrated *in vacuo* to give 6.0 g (125 %) of crude 4-(*t*-butoxycarbonylamino)cyclohexane carboxylic acid as a colorless oil which solidified upon standing.

### B. 4-(t-Butoxycarbonylamino)-N-[5-[[[5-(1,1-dimethylethyl)-2-oxazolyl]-methyl]thio]-2-thiazolyl]cyclohexylcarboxamide

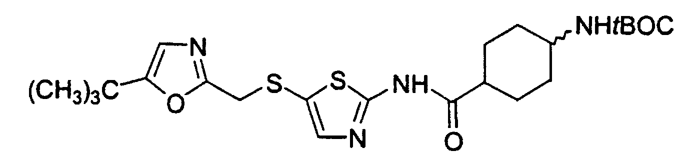

To a solution of 5 g of crude 4-(*t*-butoxycarbonylamino)cyclohexane carboxylic acid and 3.50 g (13 mmol) of 2-amino-5-[[[5-(1,1-dimethylethyl)-2-oxazolyl]methyl]thio]thiazole in 13 mL of N,N-dimethylformamide and 36 mL of methylene chloride was added 5.0 g (26 mmol) of 1-(3-dimethylaminopropyl)-3-ethylcarbodiimide hydrochloride at room temperature. The reaction mixture was stirred overnight and diluted with 100 mL of water. The aqueous layer was separated and extracted with two-150 mL portions of ethyl acetate. The combined organic phases were dried (sodium sulfate) then filtered through a pad of silica gel. The filtrate was concentrated *in vacuo* to afford an orange solid. The crude material was recrystallized (95 % ethanol) to give 4-(*t*-butoxycarbonylamino)-N-[5-[[[5-(1,1-dimethylethyl)-2-oxazolyl]methyl]thio]-2-thiazolyl]cyclohexylcarboxamide as a yellow solid. The mother liquors were also concentrated *in vacuo* to give additional 4-(*t*-butoxycarbonylamino)-N-[5-[[[5-(1,1-dimethylethyl)-2-oxazolyl]methyl]thio]-2-thiazolyl]cyclohexylcarboxamide as a brown solid.

### C. cis-4-Amino-N-[5-[[[5-(1,1-dimethylethyl)-2-oxazolyl]methyl]thio]-2-thiazolyl]cyclohexylcarboxamide hydrochloride and trans-4-Amino-N-[5-[[[5-(1,1-dimethylethyl)-2-oxazolyl]methyl]thio]-2-thiazolyl]-cyclohexylcarboxamide hydrochloride

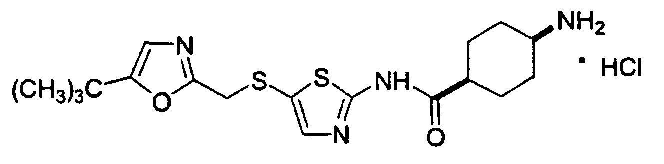

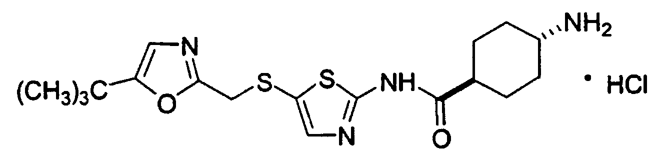

To a suspension of 4-(*t*-butoxycarbonylamino)-N-[5-[[[5-(1,1-dimethylethyl)-2-oxazolyl]methyl]thio]-2-thiazolyl]cyclohexylcarboxamide (from Part B mother liquors) suspended in 15 mL of methylene chloride was added 5 mL of trifluoroacetic acid at room temperature. The reaction mixture was stirred for 2 h then concentrated *in vacuo* to remove volatiles. The residue was diluted with water, basified with aqueous NaOH solution then the resulting aqueous solution was extracted with ethyl acetate. The combined organic extracts were dried (sodium sulfate) to give a crude cis/trans product. The crude material was purified by flash chromatography (Merck silica, 25x3 cm, 1:9 isopropylamine/ethyl acetate then 1:2:7 methanol/isopropylamine/ethyl acetate) to afford 0.74 g of the cis isomer as a yellow solid and 0.50 g of the trans isomer as a brown solid. The cis isomer was dissolved in methanol then 0.34 mL of 5N aqueous HCl was added. The solution was concentrated *in vacuo*, washed with ether, diluted with water and lyophilized to afford 0.80 g of cis-4-amino-N-[5-[[[5-(1,1-dimethylethyl)-2-oxazolyl]methyl]thio]-2-thiazolyl]cyclohexylcarboxamide hydrochloride as a yellow solid. MS: 395 [M+H]⁺; HPLC-HI 98 % at 3.17 min (YMC S5 ODS column 4.6 x 50 mm, 10-90 % aqueous methanol over 4 minutes containing 0.2 % phosphoric acid, 4 mL/min, monitoring at 220 nm). The trans isomer was dissolved in methanol then 0.24 mL of 5N aqueous HCl was added. The solution was concentrated *in vacuo*, washed with ether, diluted with water and lyophilized to afford 0.54 g of trans-4-amino-N-[5-[[[5-(1,1-dimethylethyl)-2-oxazolyl]methyl]thio]-2-thiazolyl]cyclohexylcarboxamide hydrochloride as an orange solid. MS: 395 [M+H]⁺; HPLC-HI 96 % at 3.22 min (YMC S5 ODS column 4.6 x 50 mm, 10-90 % aqueous methanol over 4 minutes containing 0.2 % phosphoric acid, 4 mL/min, monitoring at 220 nm).

### Example 10

### N-[5-[[[5-(1,1-Dimethylethyl)-2-oxazolyl]methyl]thio]-2-thiazolyl]-4-piperidinecarboxamide, monohydrochloride

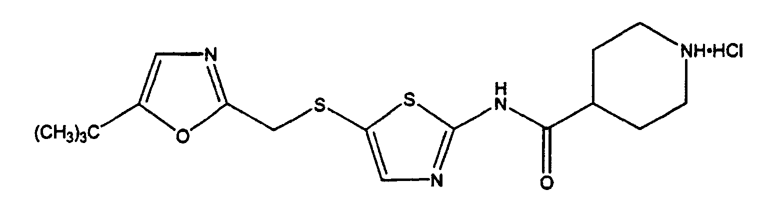

To a solution of 40 mL of absolute EtOH cooled in an ice-bath was added acetyl chloride (0.28 mL, 3.9 mmol) dropwise. The reaction mixture was allowed to warm to room temperature over 30 min then N-[5-[[[5-(1,1-dimethylethyl)-2-oxazolyl]methyl]-thio]-2-thiazolyl]-4-piperidinecarboxamide (1.50 g, 3.94 mmol, I eq) was introduced in one portion with stirring to give a thick slurry. Water (∼4 mL) was added until homogeneous then concentrated in vacuo to give a crude pale yellow solid. The crude material was recrystallized (aq EtOH) to afford the title compound (70%) as a white solid, mp 256-258°. Analysis calc'd for C17H24N4O2S2·HCl: C, 48.96; H, 6.04; N, 13.43; S, 15.38; Cl, 8.50. Found: C, 48.69; H, 5.99; N, 13.24; S, 15.27; Cl, 8.31.

### Example 11

### N-[5-[[[5-(1,1-Dimethylethyl)-2-oxazolyl]methyl]thio]-2-thiazolyl]-4-piperidinecarboxamide, monohydrobromide

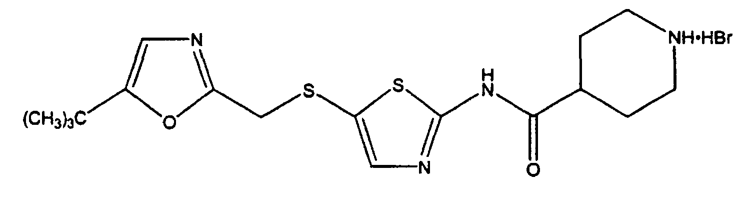

To a solution of 1M HBr in EtOH (0.5 mL) was added N-[5-[[[5-(1,1-dimethylethyl)-2-oxazolyl]methyl]thio]-2-thiazolyl]-4-piperidinecarboxamide (190 mg, 0.5 mmol, 1 eq) then cooled to -40°C overnight. The solid precipitate that formed was collected on a Buchner funnel, washed with absolute EtOH then dried under vacuum at 100°C to afford the title compound (72%) as a fine white powder, mp 235-237° C. Analysis calc'd for C17H24N4O2S2·HBr: C, 44.24; H, 5.46; N, 12.14; S, 13.89; Br, 17.31. Found: C, 44.16; H, 5.40; N, 12.12; S, 13.91; Br, 17.70.

### Example 12

### N-[5-[[[5-(1,1-Dimethylethyl)-2-oxazolyl]methyl]thio]-2-thiazolyl]-4-piperidinecarboxamide, 0.5-L-tartaric acid salt

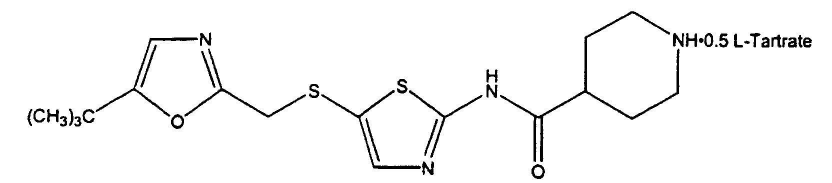

To a warm solution of N-[5-[[[5-(1,1-dimethylethyl)-2-oxazolyl]methyl]thio]-2-thiazolyl]-4-piperidinecarboxamide (1.75 g, 4.6 mmol) in absolute EtOH (70 mL) was added a solution of L-tartaric acid (345 mg, 2.3 mmol, 0.5 eq) in absolute EtOH (5 mL). A precipitate started to form after several minutes. The mixture was allowed to stand for 4 hr at room temperature then the solid precipitate was collected on a Buchner funnel, washed with absolute EtOH and dried under vacuum at 85°C for 24 hr to afford the title compound (94%) as pale yellow crystals, mp 234-236°C. Analysis calc'd for C17H24N4O2S2·0.5-L-Tartaric acid: C, 50.09; H, 5.97; N, 12.29; S, 14.07. Found: C, 49.85; H, 5.90; N, 12.12; S, 13.75.

### Example 13

### N-[5-[[[5-(1,1-Dimethylethyl)-2-oxazolyl]methyl]thio]-2-thiazolyl]-4-piperidinecarboxamide, 0.5-D-tartaric acid salt

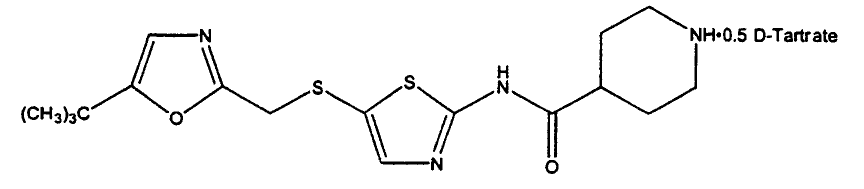

To a warm solution of N-[5-[[[5-(1,1-dimethylethyl)-2-oxazolyl]methyl]thio]-2-thiazolyl]-4-piperidinecarboxamide (1.00 g, 2.63 mmol) in absolute EtOH (40 mL) was added a solution of D-tartaric acid (198 mg, 1.32 mmol, 0.5 eq) in absolute EtOH (4 mL). A precipitate started to form after several minutes. The mixture was allowed to stand for 18 hr at room temperature then the solid precipitate was collected on a Buchner funnel, washed with absolute EtOH and dried under vacuum at 65°C for 6 hr to afford the title compound (73%) as a white solid, mp 232-233°C. Analysis calc'd for C17H24N4O2S2·0.5-D-Tartaric acid: C, 50.09; H, 5.97; N, 12.29; S, 14.07. Found: C, 49.75; H, 5.81; N, 12.04; S, 13.37.

### Example 14

### N-[5-[[[5-(1,1-Dimethylethyl)-2-oxazolyl]methyl]thio]-2-thiazolyl]-4-piperidinecarboxamide, 0.5-fumaric acid salt

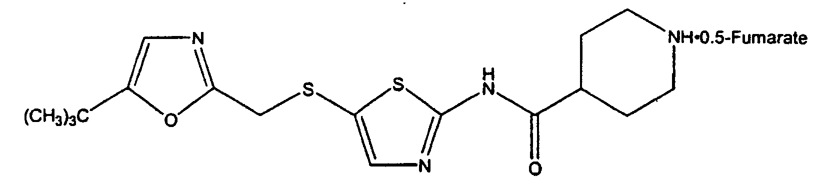

To a warm solution of N-[5-[[[5-(1,1-dimethylethyl)-2-oxazolyl]methyl]thio]-2-thiazolyl]-4-piperidinecarboxamide (1.75 g, 4.6 mmol) in absolute EtOH (100 mL) was added a solution of fumaric acid (276 mg, 2.3 mmol, 0.5 eq) in absolute EtOH (5 mL). A precipitate started to form after 10 minutes. The mixture was allowed to stand for 2 hr at room temperature then at 5°C for 16 hr. The solid precipitate which formed was collected on a Buchner funnel, washed with absolute EtOH and dried under vacuum at 65°C for 24 hr to afford the title compound (84%) as a white solid, mp 206-207° C. Analysis calc'd for C17H24N4O2S2·0.5-Fumaric acid: C, 52.04; H, 5.98; N, 12.77; S, 14.62. Found: C, 51.74; H, 5.76; N, 12.57; S, 14.19. Recrystallization (95% aq EtOH) afforded the title compound containing 1 mol EtOH (83%) as large colorless crystals, mp 212-214° C. Analysis calc'd for C17H24N4O2S2·0.5-Fumaric acid·EtOH: C, 52.05; H, 6.66; N, 11.56; S, 13.23. Found: C, 52.03; H, 6.06; N, 11.50; S, 12.99.

### Example 15

### N-[5-[[[5-(1,1-Dimethylethyl)-2-oxazolyl]methyl]thio]-2-thiazolyl]-4-piperidinecarboxamide, 0.5-succinic acid salt

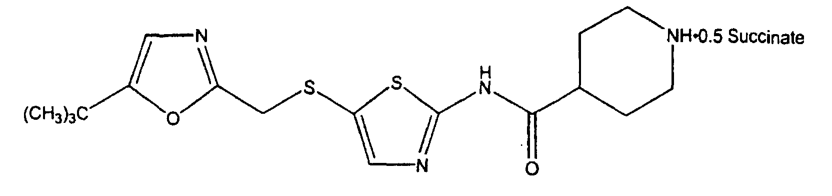

To a warm solution of N-[5-[[[5-(1,1-dimethylethyl)-2-oxazolyl]methyl]thio]-2-thiazolyl]-4-piperidinecarboxamide (50 mg, 0.13 mmol) in absolute EtOH (2 mL) was added a solution of succinic acid (7.7 mg, 0.065 mmol, 0.5 eq) in absolute EtOH (0.25 mL). A precipitate started to form after 10 minutes. The mixture was allowed to stand for 1 hr at room temperature then the precipitate was collected on a Buchner funnel, washed with absolute EtOH and dried under vacuum at 100° C for 24 hr to afford the title compound (70%) as a white solid, mp 190-192° C. Analysis calc'd for C17H24N4O2S2·0.5-Succinic acid·0.46H2O: C, 50.96; H, 6.28; N, 12.51; S, 14.32. Found: C, 50.96; H, 6.20; N, 12.49; S, 14.23.

### Example 16

### N-[5-[[[5-(1,1-Dimethylethyl)-2-oxazolyl]methyl]thio]-2-thiazolyl]-4-piperidinecarboxamide, 0.5-sulfuric acid salt

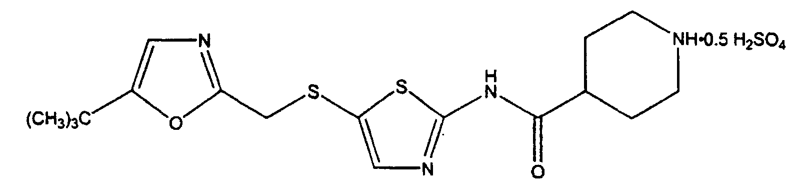

To a warm solution of N-[5-[[[5-(1,1-dimethylethyl)-2-oxazolyl]methyl]thio]-2-thiazolyl]-4-piperidinecarboxamide (50 mg, 0.13 mmol) in absolute EtOH (2 mL) was added a 1M aq solution of sulfuric acid (0.065 mL, 0.065 mmol, 0.5 eq ). A precipitate formed almost immediately. The mixture was cooled to 5° C. for 2 hr then the precipitate was collected on a Buchner funnel, washed with absolute EtOH and dried under vacuum at 100° C for 24 hr to afford the title compound (79%) as a white solid, mp 256-258° C. Analysis calc'd for C17H24N4O2S2·0.5H2SO4·0.68H2O: C, 46.22; H, 6.01; N, 12.68; S, 18.14. Found: C, 46.21; H, 5.95; N, 12.71; S, 18.23.

### Example 17

### N-[5-[[[5-(1,1-Dimethylethyl)-2-oxazolyl]methyl]thio]-2-thiazolyl]-4-piperidinecarboxamide, 0.5-citric acid salt

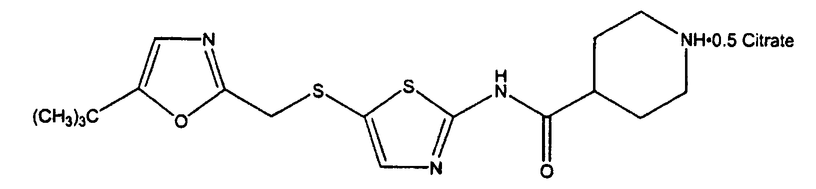

To a warm solution of N-[5-[[[5-( 1,1-dimethylethyl)-2-oxazolyl]methyl]thio]-2-thiazolyl]-4-piperidinecarboxamide (50 mg, 0.13 mmol) in absolute EtOH (2 mL) was added a solution of citric acid (8.3 mg, 0.043 mmol, 0.33 eq ). The solution was cooled to 5° C for 18 hr then the precipitate which formed was collected on a Buchner funnel, washed with absolute EtOH and dried under vacuum at 100° C for 24 hr to afford the title compound (68%) as a white solid, mp 214-216° C. Analysis calc'd for C17H24N4O2S2·0.5-Citric acid·0.10H2O: C, 50.21; H, 5.94; N, 11.71; S, 13.40. Found: C, 50.21; H, 6.01; N, 11.83; S, 13.44.

### Example 18

### N-[5-[[[5-(1,1-Dimethylethyl)-2-oxazolyl]methyl]thio]-2-thiazolyl]-4-piperidinecarboxamide, methanesulfonic acid salt

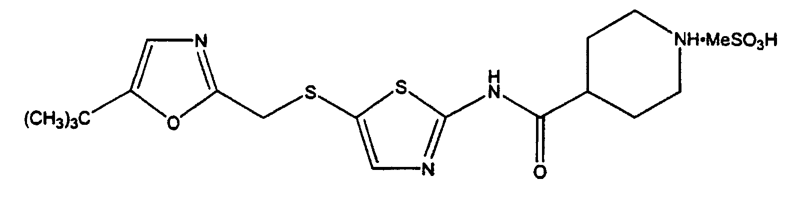

To a slurry of N-[5-[[[5-(1,1-dimethylethyl)-2-oxazolyl]methyl]thio]-2-thiazolyl]-4-piperidinecarboxamide (100 mg, 0.26 mmol) in isopropyl alcohol (0.75 mL) was added methanesulfonic acid (0.017 mL, 0.26 mmol, 1 eq ). The slurry was heated to 70° C to give a clear solution then methyl t-butyl ether (1.5 mL) was added. Within 15 minutes a precipitate formed. The resulting mixture was stirred at 55° C for 2 hr then at room temperature for 14 hr. The precipitate which formed was collected by filtration then dried under vacuum at 50° C for 14 hr to afford the title compound (85%) as a colorless powder, mp 105° C. Analysis calc'd for C17H24N4O2S2·MSA·H2O: C, 43.70; H, 6.11; N, 11.32; S, 19.44. Found: C, 43.53; H, 6.14; N, 11.15; S, 19.15.

### Example 19

### N-[5-[[[5-(1,1-Dimethylethyl)-2-oxazolyl]methyl]thio]-2-thiazolyl]-4-piperidinecarboxamide, 0.5-D,L-malic acid salt

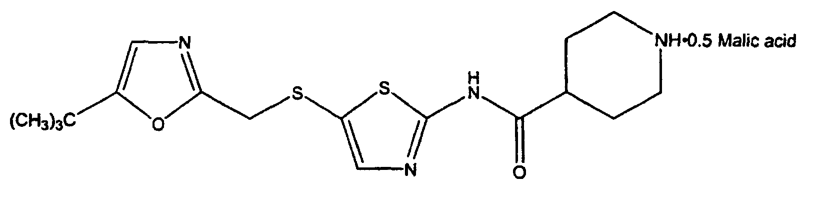

To a solution of N-[5-[[[5-(1,1-dimethylethyl)-2-oxazolyl]methyl]thio]-2-thiazolyl]-4-piperidinecarboxamide (100 mg, 0.26 mmol) in isopropyl alcohol (0.80 mL) was added slowly at 70° C a solution of D,L-malic acid (35 mg, 0.13 mmol, 0.5 eq ) in isopropyl alcohol (0.3 mL). A precipitate formed immediately. The resulting mixture was stirred at 55° C for 2 hr then at room temperature for 14 hr. The precipitate was collected by filtration then dried under vacuum at 50° C for 14 hr to afford the title compound (75%) as a colorless powder, mp 216° C. Analysis calc'd for C17H24N4O2S2·0.5-C4H6O5·H2O: C, 50.98; H, 6.08; N, 12.51; S, 14.32. Found: C, 50.55; H, 6.17; N, 12.29; S, 14.05.

## Claims

1. A compound of formula I: 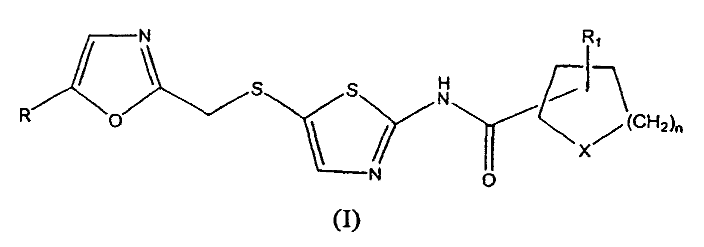 and enantiomers, diastereomers and pharmaceutically acceptable salts thereof wherein
R is t-butyl;
R₁ is hydrogen or alkyl;
X is NR₂ or CHNR₂R₃;
R₂ and R₃ are each independently hydrogen, alkyl, substituted alkyl, cycloalkyl or substituted cycloalkyl; and
n is 0, 1, 2 or 3.

2. The compound according to claim I wherein
R is t-butyl;
R₁ is hydrogen;
X is NR₂ or CHNR₂R₃;
R₂ and R₃ are each independently hydrogen, alkyl, substituted alkyl or cycloalkyl; and
n is 2.

3. The compound according to claim 1 of formula Ia: 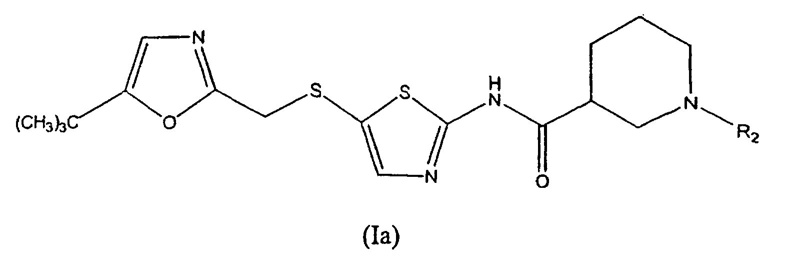 and enantiomers, diastereomers and pharmaceutically acceptable salts thereof wherein R₂ is hydrogen, alkyl, substituted alkyl or cycloalkyl.

4. The compound according to Claim 1 of formula Ib 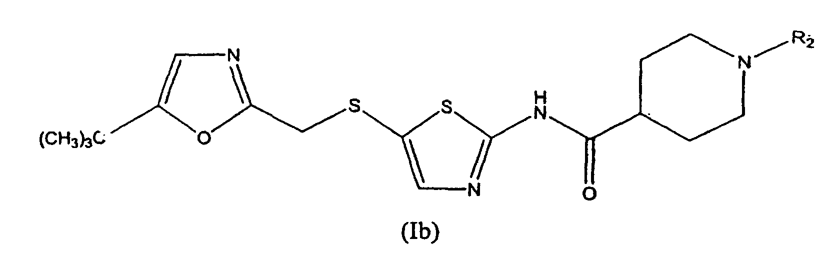 and enantiomers, diastereomers and pharmaceutically acceptable salts thereof wherein R₂ is hydrogen, alkyl, substituted alkyl or cycloalkyl.

5. The compound according to Claim 1 of formula Ic 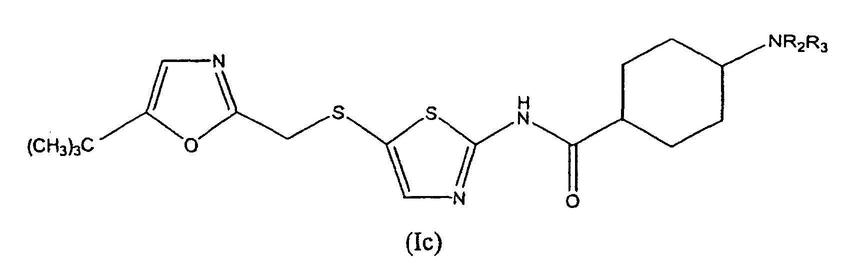 and enantiomers, diasteromers and pharmaceutically acceptable salts thereof wherein R₂ and R₃ are each independently hydrogen, alkyl, substituted alkyl or cycloalkyl.

6. The compound according to Claim 1 selected from the group consisting of
N-[5-[[[5-(1,1-dimethylethyl)-2-oxazolyl]methyl]thio]-2-thiazolyl]-4-piperidinecarboxamide;
(±)-N-[5-[[[5-(1,1-dimethylethyl)-2-oxazolyl]methyl]thio]-2-thiazolyl]-3-piperidinecarboxamide;
(±)-1-(2,3-dihydroxypropyl)-N-[5-[[[5-(1,1-dimethylethyl)-2-oxazolyl]-methyl]thio]-2-thiazolyl]-4-piperidinecarboxamide;
N-[5-[[[5-(1,1-dimethylethyl)-2-oxazolyl]methyl]thio]-2-thiazolyl]-1-(1-methylethyl)-4-piperidinecarboxamide;
1-cyclopropyl-N-[5-[[[5-(1,1-dimethylethyl)-2-oxazolyl]methyl]thio]-2-thiazolyl]-4-piperidinecarboxamide;
N-[5-[[[5-(1,1-dimethylethyl)-2-oxazolyl]methyl]thio]-2-thiazolyl]-1-(2-hydroxyethyl)-4-piperidinecarboxamide;
(R)-N-[5-[[[5-(1,1-dimethylethyl)-2-oxazolyl]methyl]thio]-2-thiazolyl]-3-piperidinecarboxamide;
(S)-N-[5-[[[5-(1,1-dimethylethyl)-2-oxazolyl]methyl]thio]-2-thiazolyl]-3-piperidinecarboxamide;
cis-4-amino-N-[5-[[[5-(1,1-dimethylethyl)-2-oxazolyl]methyl]thio]-2-thiazolyl]cyclohexylcarboxamide; and
trans-4-amino-N-[5-[[[5-(1,1-dimethylethyl)-2-oxazolyl]methyl]thio]-2-thiazolyl]cyclohexylcarboxamide; and
pharmaceutically acceptable salts thereof.

7. The compound according to claim 1, wherein the compound is N-[5-[[[5-(1,1-dimethylethyl)-2-oxazolyl]methyl]thio]-2-thiazolyl]-4-piperidinecarboxamide and pharmaceutically acceptable salts thereof.

8. The compound according to claim 1, wherein the compound is (±)-N-[5-[[[5-(1,1-dimethylethyl)-2-oxazolyl]methyl]thio]-2-thiazolyl]-3-piperidinecarboxamide and pharmaceutically acceptable salts thereof.

9. The compound according to claim 1, wherein the compound is (R)-N-[5-[[[5-(1,1-dimethylethyl)-2-oxazolyl]methyl]thio]-2-thiazolyl]-3-piperidinecarboxamide and pharmaceutically acceptable salts thereof.

10. The compound according to claim 1, wherein the compound is (S)-N-[5-[[[5-(1,1-dimethylethyl)-2-oxazolyl]methyl]thio]-2-thiazolyl]-3-piperidinecarboxamide and pharmaceutically acceptable salts thereof.

11. The compound according to claim 1, wherein the compound is cis-4-amino-N-[5-[[[5-(1,1-dimethylethyl)-2-oxazolyl]-methyl]thio]-2-thiazolyl]cyclohexylcarboxamide and pharmaceutically acceptable salts thereof.

12. The compound according to claim 1, wherein the compound is trans-4-amino-N-[5-[[[5-(1,1-dimethylethyl)-2-oxazolyl]-methyl]thio]-2-thiazolyl]cyclohexylcarboxamide and pharmaceutically acceptable salts thereof.

13. A pharmaceutical composition which comprises a compound according to any of Claims 1 to 12 and a pharmaceutically acceptable carrier.

14. A pharmaceutical composition which comprises a compound according to any of Claims 1 to 12 in combination with a pharmaceutically acceptable carrier and an anti-cancer agent formulated as a fixed dose.

15. A pharmaceutical composition which comprises a compound according to any of Claims 1 to 12 in combination with a pharmaceutically acceptable carrier and a modulator of p53 transactivation formulated as a fixed dose.

16. Use of a compound according to any of Claims 1 to 12 in the manufacture of a medicament for the treatment of a condition treatable through the modulation of apoptosis.

17. Use of a compound according to any of Claims 1 to 12 in the manufacture of a medicament for the treatment of a condition treatable through the inhibition of protein kinases.

18. Use of a compound according to any of Claims 1 to 12 in the manufacture of a medicament for the treatment of a condition treatable through the inhibition of cyclin dependent kinases.

19. Use of a compound according to any of Claims 1 to 12 in the manufacture of a medicament for the treatment of a condition treatable through the inhibition of cdc2 (cdkl).

20. Use of a compound according to any of Claims 1 to 12 in the manufacture of a medicament for the treatment of a condition treatable through the inhibition of cdk2.

21. Use of a compound according to any of Claims 1 to 12 in the manufacture of a medicament for the treatment of a condition treatable through the inhibition of cdk3.

22. Use of a compound according to any of Claims 1 to 12 in the manufacture of a medicament for the treatment of a condition treatable through the inhibition of cdk4.

23. Use of a compound according to any of Claims 1 to 12 in the manufacture of a medicament for the treatment of a condition treatable through the inhibition of cdk5.

24. Use of a compound according to any of Claims 1 to 12 in the manufacture of a medicament for the treatment of a condition treatable through the inhibition of cdk6.

25. Use of a compound according to any of Claims 1 to 12 in the manufacture of a medicament for the treatment of a condition treatable through the inhibition of cdk7.

26. Use of a compound according to any of Claims 1 to 12 in the manufacture of a medicament for the treatment of a condition treatable through the inhibition of cdk8.

27. Use of a composition according to any of claims 13 to 15 in the manufacture of a medicament for the treatment of proliferative diseases.

28. Use of a composition according to any of claims 13 to 15 in the manufacture of a medicament for the treatment of cancer.

29. Use of a composition according to any of claims 13 to 15 in the manufacture of a medicament for the treatment of inflammation, inflammatory bowel disease or transplantation rejection.

30. Use of a composition according to any of claims 13 to 15 in the manufacture of a medicament for the treatment of arthritis.

31. Use of a compound according to any of Claims 1 to 12 in the manufacture of a medicament for the treatment of a cyclin dependent kinase-associated disorder.

32. A compound according to any of claims 1 to 12 wherein said pharmaceutically acceptable salt is selected from the group consisting of hydrochloride, dihydrochloride, sulfate, trifluoroacetate, mixture of trifluoroacetate and hydrochloride, tartarate, fumarate, succinate, maleate, citrate, methanesulfonate, bromate and iodate salts.

33. A pharmaceutical composition according to any of claims 13 to 15 wherein said pharmaceutically acceptable salt is selected from the group consisting of hydrochloride, dihydrochloride, sulfate, trifluoroacetate, mixture of trifluoroacetate and hydrochloride, tartarate, fumarate, succinate, maleate, citrate, methanesulfonate, bromate and iodate salts.

34. Use according to any of claims 16 to 32 wherein said pharmaceutically acceptable salt of said compound is selected from the group consisting of hydrochloride, dihydrochloride, sulfate, trifluoroacetate, mixture of trifluoroacetate and hydrochloride, tartarate, fumarate, succinate, maleate, citrate, methanesulfonate, bromate and iodate salts.

35. A compound according to claim 32 wherein said pharmaceutically acceptable salt is tartarate.

36. A compound according to claim 35 wherein said pharmaceutically acceptable salt is tartarate and wherein the compound shows the structure of formula (Ib) of claim 4.

37. A compound according to claim 36 wherein said compound is a tartaric acid salt of N-[5-[[[5-(1,1-dimethylethyl)-2-oxazolyl]methyl]thio]-2-thiazolyl]-4-piperidinecarboxamide.

## Patentansprüche

1. Verbindung der Formel I: 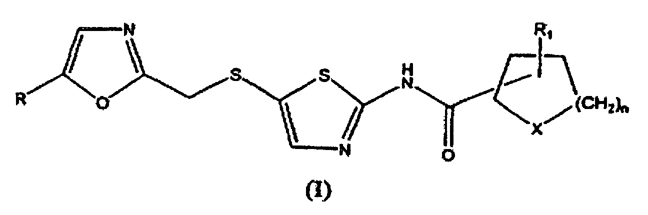 und Enantiomere, Diastereomere und pharmazeutisch verträgliche Salze davon, wobei
R t-Butyl ist;
R₁ ein Wasserstoffatom oder Alkylrest ist;
X NR₂ oder CHNR₂R₃ ist;
R₂ und R₃ jeweils unabhängig ein Wasserstoffatom, Alkylrest, substituierter Alkylrest, Cycloalkylrest oder substituierter Cycloalkylrest sind; und
n gleich 0, 1, 2 oder 3 ist.

2. Verbindung gemäß Anspruch 1, wobei
R t-Butyl ist;
R₁ ein Wasserstoffatom ist;
X NR₂ oder CHNR₂R₃ ist;
R₂ und R₃ jeweils unabhängig ein Wasserstoffatom, Alkylrest, substituierter Alkylrest oder Cycloalkylrest sind; und
n gleich 2 ist.

3. Verbindung gemäß Anspruch 1 der Formel Ia: 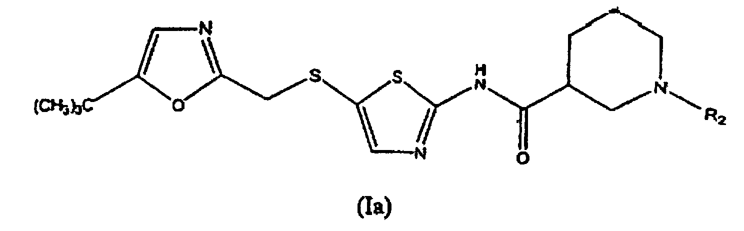 und Enantiomere, Diastereomere und pharmazeutisch verträgliche Salze davon, wobei R₂ ein Wasserstoffatom, Alkylrest, substituierter Alkylrest oder Cycloalkylrest ist.

4. Verbindung gemäß Anspruch 1 der Formel Ib: 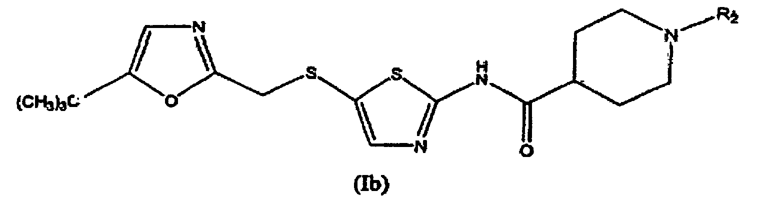 und Enantiomere, Diastereomere und pharmazeutisch verträgliche Salze davon, wobei R₂ ein Wasserstoffatom, Alkylrest, substituierter Alkylrest oder Cycloalkylrest ist.

5. Verbindung gemäß Anspruch 1 der Formel Ic: 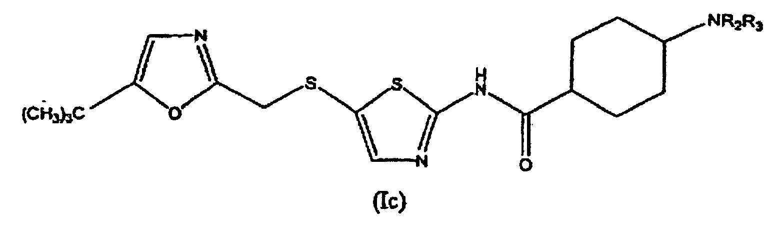 und Enantiomere, Diastereomere und pharmazeutisch verträgliche Salze davon, wobei R₂ und R₃ jeweils unabhängig ein Wasserstoffatom, Alkylrest, substituierter Alkylrest oder Cycloalkylrest sind.

6. Verbindung gemäß Anspruch 1, ausgewählt aus
N-[5-[[[5-(1,1-Dimethylethyl)-2-oxazolyl]methyl]thio]-2-thiazolyl]-4-piperidincarboxamid;
(±)-N-[5-[[[5-(1,1-Dimethylethyl)-2-oxazolyl]methyl]thio]-2-thiazolyl]-3-piperidincarboxamid;
(±)-1-(2,3-Dihydroxypropyl)-N-[5-[[[5-(1,1-dimethylethyl)-2-oxazolyl]methyl]thio]-2-thiazolyl]-4-piperidincarboxamid;
N-[5-[[[5-(1,1-Dimethylethyl)-2-oxazolyl]methyl]thio]-2-thiazolyl]-1-(1-methylethyl)-4-piperidincarboxamid;
1-Cyclopropyl-N-[5-[[[5-(1,1-dimethylethyl)-2-oxazolyl]methyl]thio]-2-thiazolyl]-4-piperidincarboxamid;
N-[5-[[[5-(1,1-Dimethylethyl)-2-oxazolyl]methyl]thio]-2-thiazolyl]-1-(2-hydroxyethyl)-4-piperidincarboxamid;
(R)-N-[5-[[[5-(1,1-Dimethylethyl)-2-oxazolyl]methyl]thio]-2-thiazolyl]-3-piperidincarboxamid;
(S)-N-[5-[[[5-(1,1-Dimethylethyl)-2-oxazolyl]methyl]thio]-2-thiazolyl]-3-piperidincarboxamid;
cis-4-Amino-N-[5-[[[5-(1,1-dimethylethyl)-2-oxazolyl]methyl]thio]-2-thiazolyl]-cyclohexylcarboxamid; und
trans-4-Amino-N-[5-[[[5-(1,1-dimethylethyl)-2-oxazolyl]methyl]thio]-2-thiazolyl]-cyclohexylcarboxamid; und
pharmazeutisch verträglichen Salzen davon.

7. Verbindung gemäß Anspruch 1, wobei die Verbindung N-[5-[[[5-(1,1-Dimethylethyl)-2-oxazolyl]methyl]thio]-2-thiazolyl]-4-piperidincarboxamid ist und pharmazeutisch verträgliche Salze davon.

8. Verbindung gemäß Anspruch 1, wobei die Verbindung (±)-N-[5-[[[5-(1,1-Dimethylethyl)-2-oxazolyl]methyl]thio]-2-thiazolyl]-3-piperidincarboxamid ist und pharmazeutisch verträgliche Salze davon.

9. Verbindung gemäß Anspruch 1, wobei die Verbindung (R)-N-[5-[[[5-(1,1-Dimethylethyl)-2-oxazolyl]methyl]thio]-2-thiazolyl]-3-piperidincarboxamid ist und pharmazeutisch verträgliche Salze davon.

10. Verbindung gemäß Anspruch 1, wobei die Verbindung (S)-N-[5-[[[5-(1,1-Dimethylethyl)-2-oxazolyl]methyl]thio]-2-thiazolyl]-3-piperidincarboxamid ist und pharmazeutisch verträgliche Salze davon.

11. Verbindung gemäß Anspruch 1, wobei die Verbindung cis-4-Amino-N-[5-[[[5-(1,1-dimethylethyl)-2-oxazolyl]methyl]thio]-2-thiazolyl]cyclohexylcarboxamid ist und pharmazeutisch verträgliche Salze davon.

12. Verbindung gemäß Anspruch 1, wobei die Verbindung trans-4-Amino-N-[5-[[[5-(1,1-dimethylethyl)-2-oxazolyl]methyl]thio]-2-thiazolyl]cyclohexylcarboxamid ist und pharmazeutisch verträgliche Salze davon.

13. Arzneimittel, umfassend eine Verbindung gemäß einem der Ansprüche 1 bis 12 und einen pharmazeutisch verträglichen Träger.

14. Arzneimittel, umfassend eine Verbindung gemäß einem der Ansprüche 1 bis 12 in Kombination mit einem pharmazeutisch verträglichen Träger und einem Antikrebsmittel, formuliert als feste Dosis.

15. Arzneimittel, umfassend eine Verbindung gemäß einem der Ansprüche 1 bis 12 in Kombination mit einem pharmazeutisch verträglichen Träger und einem Modulator der p53 Transaktivierung, formuliert als feste Dosis.

16. Verwendung einer Verbindung gemäß einem der Ansprüche 1 bis 12 zur Herstellung eines Medikaments zur Behandlung eines Zustands, der durch die Modulation der Apoptosis behandelbar ist.

17. Verwendung einer Verbindung gemäß einem der Ansprüche 1 bis 12 zur Herstellung eines Medikaments zur Behandlung eines Zustands, der durch die Hemmung von Proteinkinasen behandelbar ist.

18. Verwendung einer Verbindung gemäß einem der Ansprüche 1 bis 12 zur Herstellung eines Medikaments zur Behandlung eines Zustands, der durch die Hemmung von cyclinabhängigen Kinasen behandelbar ist.

19. Verwendung einer Verbindung gemäß einem der Ansprüche 1 bis 12 zur Herstellung eines Medikaments zur Behandlung eines Zustands, der durch die Hemmung von cdc2 (cdkl) behandelbar ist.

20. Verwendung einer Verbindung gemäß einem der Ansprüche 1 bis 12 zur Herstellung eines Medikaments zur Behandlung eines Zustands, der durch die Hemmung von cdk2 behandelbar ist.

21. Verwendung einer Verbindung gemäß einem der Ansprüche 1 bis 12 zur Herstellung eines Medikaments zur Behandlung eines Zustands, der durch die Hemmung von cdk3 behandelbar ist.

22. Verwendung einer Verbindung gemäß einem der Ansprüche 1 bis 12 zur Herstellung eines Medikaments zur Behandlung eines Zustands, der durch die Hemmung von cdk4 behandelbar ist.

23. Verwendung einer Verbindung gemäß einem der Ansprüche 1 bis 12 zur Herstellung eines Medikaments zur Behandlung eines Zustands, der durch die Hemmung von cdk5 behandelbar ist.

24. Verwendung einer Verbindung gemäß einem der Ansprüche 1 bis 12 zur Herstellung eines Medikaments zur Behandlung eines Zustands, der durch die Hemmung von cdk6 behandelbar ist.

25. Verwendung einer Verbindung gemäß einem der Ansprüche 1 bis 12 zur Herstellung eines Medikaments zur Behandlung eines Zustands, der durch die Hemmung von cdk7 behandelbar ist.

26. Verwendung einer Verbindung gemäß einem der Ansprüche 1 bis 12 zur Herstellung eines Medikaments zur Behandlung eines Zustands, der durch die Hemmung von cdk8 behandelbar ist.

27. Verwendung eines Arzneimittels gemäß einem der Ansprüche 13 bis 15 zur Herstellung eines Medikaments zur Behandlung von proliferativen Krankheiten.

28. Verwendung eines Arzneimittels gemäß einem der Ansprüche 13 bis 15 zur Herstellung eines Medikaments zur Behandlung von Krebs.

29. Verwendung eines Arzneimittels gemäß einem der Ansprüche 13 bis 15 zur Herstellung eines Medikaments zur Behandlung einer Entzündung, einer entzündlichen Darmerkrankung oder Transplantationsabstoßung.

30. Verwendung eines Arzneimittels gemäß einem der Ansprüche 13 bis 15 zur Herstellung eines Medikaments zur Behandlung von Arthritis.

31. Verwendung einer Verbindung gemäß einem der Ansprüche 1 bis 12 zur Herstellung eines Medikaments zur Behandlung einer mit einer cyclin-abhängigen Kinase assoziierten Störung.

32. Verbindung gemäß einem der Ansprüche 1 bis 12, wobei das pharmazeutisch verträgliche Salz aus Hydrochlorid-, Dihydrochlorid-, Sulfat-, Trifluoracetat-, einem Gemisch aus Trifluoracetat- und Hydrochlorid-, Tartrat-, Fumarat-, Succinat-, Maleat-, Citrat-, Methansulfonat-, Bromat- und Iodatsalzen ausgewählt ist.

33. Arzneimittel gemäß einem der Ansprüche 13 bis 15, wobei das pharmazeutisch verträgliche Salz aus Hydrochlorid-, Dihydrochlorid-, Sulfat-, Trifluoracetat-, einem Gemisch aus Trifluoracetat- und Hydrochlorid-, Tartrat-, Fumarat-, Succinat-, Maleat-, Citrat-, Methansulfonat-, Bromat- und Iodatsalzen ausgewählt ist.

34. Verwendung gemäß einem der Ansprüche 16 bis 32, wobei das pharmazeutisch verträgliche Salz der Verbindung aus Hydrochlorid-, Dihydrochlorid-, Sulfat-, Trifluoracetat-, einem Gemisch aus Trifluoracetat- und Hydrochlorid-, Tartrat-, Fumarat-, Succinat-, Maleat-, Citrat-, Methansulfonat-, Bromat- und Iodatsalzen ausgewählt ist.

35. Verbindung gemäß Anspruch 32, wobei das pharmazeutisch verträgliche Salz Tartrat ist.

36. Verbindung gemäß Anspruch 35, wobei das pharmazeutisch verträgliche Salz Tartrat ist und wobei die Verbindung die Struktur der Formel (Ib) des Anspruchs 4 zeigt.

37. Verbindung gemäß Anspruch 36, wobei die Verbindung ein Weinsäuresalz von N-[5-[[[5-(1,1-Dimethylethyl)-2-oxazolyl]methyl]thio]-2-thiazolyl]-4-piperidincarboxamid ist.

## Revendications

1. Composé de formule I : 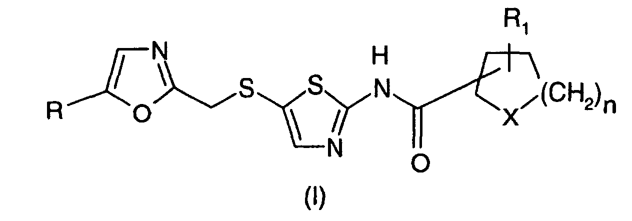 et ses énantiomères, diastéréoisomères et sels pharmaceutiquement acceptables où R est t-butyle ;
R₁ est l'hydrogène ou alkyle ;
X est NR₂ ou CHNR₂R₃ ;
R₂ et R₃ sont chacun indépendamment l'hydrogène, alkyle, alkyle substitué, cycloalkyle ou cycloalkyle substitué ; et
n est 0, 1, 2 ou 3.

2. Composé selon la revendication 1 où
R est t-butyle ;
R₁ est l'hydrogène ;
X est NR₂ ou CHNR₂R₃ ;
R₂ et R₃ sont chacun indépendamment l'hydrogène, alkyle, alkyle substitué ou cycloalkyle ; et
n est 2.

3. Composé selon la revendication 1 de formule Ia : 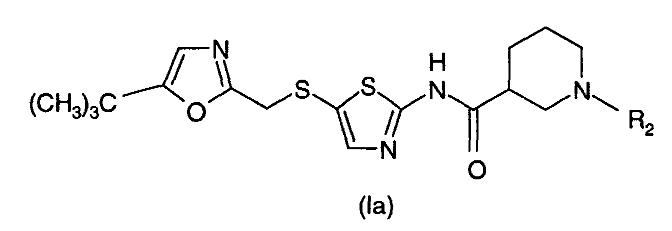 et ses énantiomères, diastéréoisomères et sels pharmaceutiquement acceptables où R₂ est l'hydrogène, alkyle, alkyle substitué ou cycloalkyle.

4. Composé selon la revendication 1 de formule Ib 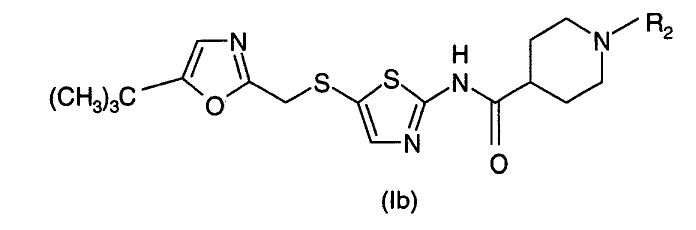 et ses énantiomères, diastéréoisomères et sels pharmaceutiquement acceptables où R₂ est l'hydrogène, alkyle, alkyle substitué ou cycloalkyle.

5. Composé selon la revendication 1 de formule Ic 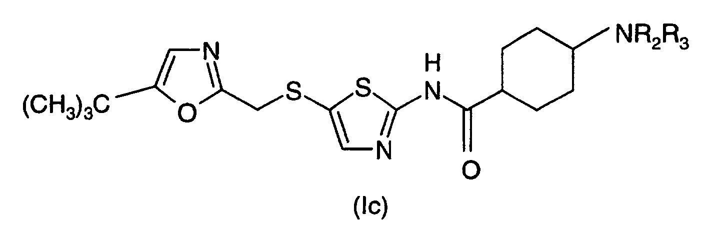 et ses énantiomères, diastéréoisomères et sels pharmaceutiquement acceptables où R₂ et R₃ sont chacun indépendamment l'hydrogène, alkyle, alkyle substitué ou cycloalkyle.

6. Composé selon la revendication 1 choisi dans le groupe consistant en
le N-[5-[[[5-(1,1-diméthyléthyl)-2-oxazolyl]méthyl]thio]-2-thiazolyl]-4-pipéridinecarboxamide ;
le (±)-N-[5-[[[5-(1,1-diméthyléthyl)-2-oxazolyl]méthyl]thio]-2-thiazolyl]-3-pipéridinecarboxamide ;
le (±)-1-(2,3-dihydroxypropyl)-N-[5-[[[5-(1,1-diméthyléthyl)-2-oxazolyl]-méthyl]thio]-2-thiazolyl]-4-pipéridinecarboxamide ;
le N-[5-[[[5-(1,1-diméthyléthyl)-2-oxazolyl]méthyl]thio]-2-thiazolyl]-1-(1-méthyléthyl)-4-pipéridinecarboxamide ;
le 1-cyclopropyl-N-[5-[[[5-(1,1-diméthyléthyl)-2-oxazolyl]méthyl]thio]-2-thiazolyl]-4-pipéridinecarboxamide ;
le N-[5-[[[5-(1,1-diméthyléthyl)-2-oxazolyl]méthyl]thio]-2-thiazolyl]-1-(2-hydroxyéthyl)-4-pipéridinecarboxamide ;
le (R)-N-[5-[[[5-(1,1-diméthyléthyl)-2-oxazolyl]méthyl]thio]-2-thiazolyl]-3-pipéridinecarboxamide ;
le (S)-N-[5-[[[5-(1,1-diméthyléthyl)-2-oxazolyl]méthyl]thio]-2-thiazolyl]-3-pipéridinecarboxamide ;
le cis-4-amino-N-[5-[[[5-(1,1-diméthyléthyl)-2-oxazolyl]méthyl]thio]-2-thiazolyl]cyclohexylcarboxamide ; et
le trans-4-amino-N-[5-[[[5-(1,1-diméthyléthyl)-2-oxazolyl]méthyl]thio]-2-thiazolyl]cyclohexylcarboxamide ;
et ses sels pharmaceutiquement acceptables.

7. Composé selon la revendication 1 où le composé est le N-[5-[[[5-(1,1-diméthyléthyl)-2-oxazolyl]méthyl]thio]-2-thiazolyl]-4-pipéridinecarboxamide et ses sels pharmaceutiquement acceptables.

8. Composé selon la revendication 1 où le composé est le (±)-N-[5-[[[5-(1,1-diméthyléthyl)-2-oxazolyl]méthyl]thio]-2-thiazolyl]-3-pipéridinecarboxamide et ses sels pharmaceutiquement acceptables.

9. Composé selon la revendication 1 où le composé est le (R)-N-[5-[[[5-(1,1-diméthyléthyl)-2-oxazolyl]méthyl]thio]-2-thiazolyl]-3-pipéridinecarboxamide et ses sels pharmaceutiquement acceptables.

10. Composé selon la revendication 1 où le composé est le (S)-N-[5-[[[5-(1,1-diméthyléthyl)-2-oxazolyl]méthyl]thio]-2-thiazolyl]-3-pipéridinecarboxamide et ses sels pharmaceutiquement acceptables.

11. Composé selon la revendication 1 où le composé est le cis-4-amino-N-[5-[[[5-(1,1-diméthyléthyl)-2-oxazolyl]méthyl]thio]-2-thiazolyl]-cyclohexylcarboxamide et ses sels pharmaceutiquement acceptables.

12. Composé selon la revendication 1 où le composé est le trans-4-amino-N-[5-[[[5-(1,1-diméthyléthyl)-2-oxazolyl]méthyl]thio]-2-thiazolyl]cyclohexylcarboxamide et ses sels pharmaceutiquement acceptables.

13. Composition pharmaceutique qui comprend un composé selon l'une quelconque des revendications 1 à 12 et un support pharmaceutiquement acceptable.

14. Composition pharmaceutique qui comprend un composé selon l'une quelconque des revendications 1 à 12 en combinaison avec un support pharmaceutiquement acceptable et un agent anticancéreux formulée sous forme d'une dose fixée.

15. Composition pharmaceutique qui comprend un composé selon l'une quelconque des revendications 1 à 12 en combinaison avec un support pharmaceutiquement acceptable et un modulateur de la transactivation de p53 formulée sous forme d'une dose fixée.

16. Utilisation d'un composé selon l'une quelconque des revendications 1 à 12 dans la fabrication d'un médicament pour le traitement d'une affection qui peut être traitée par la modulation de l'apoptose.

17. Utilisation d'un composé selon l'une quelconque des revendications 1 à 12 dans la fabrication d'un médicament pour le traitement d'une affection qui peut être traitée par l'inhibition de protéine kinases.

18. Utilisation d'un composé selon l'une quelconque des revendications 1 à 12 dans la fabrication d'un médicament pour le traitement d'une affection qui peut être traitée par l'inhibition de kinases dépendantes de cyclines.

19. Utilisation d'un composé selon l'une quelconque des revendications 1 à 12 dans la fabrication d'un médicament pour le traitement d'une affection qui peut être traitée par l'inhibition de cdc2 (cdkl).

20. Utilisation d'un composé selon l'une quelconque des revendications 1 à 12 dans la fabrication d'un médicament pour le traitement d'une affection qui peut être traitée par l'inhibition de cdk2.

21. Utilisation d'un composé selon l'une quelconque des revendications 1 à 12 dans la fabrication d'un médicament pour le traitement d'une affection qui peut être traitée par l'inhibition de cdk3.

22. Utilisation d'un composé selon l'une quelconque des revendications 1 à 12 dans la fabrication d'un médicament pour le traitement d'une affection qui peut être traitée par l'inhibition de cdk4.

23. Utilisation d'un composé selon l'une quelconque des revendications 1 à 12 dans la fabrication d'un médicament pour le traitement d'une affection qui peut être traitée par l'inhibition de cdk5.

24. Utilisation d'un composé selon l'une quelconque des revendications 1 à 12 dans la fabrication d'un médicament pour le traitement d'une affection qui peut être traitée par l'inhibition de cdk6.

25. Utilisation d'un composé selon l'une quelconque des revendications 1 à 12 dans la fabrication d'un médicament pour le traitement d'une affection qui peut être traitée par l'inhibition de cdk7.

26. Utilisation d'un composé selon l'une quelconque des revendications 1 à 12 dans la fabrication d'un médicament pour le traitement d'une affection qui peut être traitée par l'inhibition de cdk8.

27. Utilisation d'une composition selon l'une quelconque des revendications 13 à 15 dans la fabrication d'un médicament pour le traitement des maladies prolifératives.

28. Utilisation d'une composition selon l'une quelconque des revendications 13 à 15 dans la fabrication d'un médicament pour le traitement du cancer.

29. Utilisation d'une composition selon l'une quelconque des revendications 13 à 15 dans la fabrication d'un médicament pour le traitement de l'inflammation, des affections intestinales inflammatoires non spécifiques ou du rejet des greffes.

30. Utilisation d'une composition selon l'une quelconque des revendications 13 à 15 dans la fabrication d'un médicament pour le traitement de l'arthrite.

31. Utilisation d'un composé selon l'une quelconque des revendications 1 à 12 dans la fabrication d'un médicament pour le traitement d'un trouble associé à des kinases dépendantes de cyclines.

32. Composé selon l'une quelconque des revendications 1 à 12 où ledit sel pharmaceutiquement acceptable est choisi dans le groupe consistant en les sels chlorhydrate, dichlorhydrate, sulfate, trifluoroacétate, mélange de trifluoroacétate et de chlorhydrate, tartrate, fumarate, succinate, maléate, citrate, méthanesulfonate, bromate et iodate.

33. Composition pharmaceutique selon l'une quelconque des revendications 13 à 15 où ledit sel pharmaceutiquement acceptable est choisi dans le groupe consistant en les sels chlorhydrate, dichlorhydrate, sulfate, trifluoroacétate, mélange de trifluoroacétate et de chlorhydrate, tartrate, fumarate, succinate, maléate, citrate, méthanesulfonate, bromate et iodate.

34. Utilisation selon l'une quelconque des revendications 16 à 32 où ledit sel pharmaceutiquement acceptable dudit composé est choisi dans le groupe consistant en les sels chlorhydrate, dichlorhydrate, sulfate, trifluoroacétate, mélange de trifluoroacétate et de chlorhydrate, tartrate, fumarate, succinate, maléate, citrate, méthanesulfonate, bromate et iodate.

35. composé selon la revendication 32 où ledit sel pharmaceutiquement acceptable est le tartrate.

36. Composé selon la revendication 35 où ledit sel pharmaceutiquement acceptable est le tartrate et où le composé présente la structure de la formule (Ib) de la revendication 4.

37. Composé selon la revendication 36 où ledit composé est un sel d'acide tartrique et de N-[5-[[[5-(1,1-diméthyléthyl)-2-oxazolyl]-méthyl]thio]-2-thiazolyl]-4-pipéridinecarboxamide.
